# EUROPEAN PATENT SPECIFICATION

(11) **EP 2 603 797 B1**
(45) Date of publication and mention of the grant of the patent: **05.04.2017**
(21) Application number: 11741573.7
(22) Date of filing: 10.08.2011
(51) Int. Cl.: G01N 33/68

(54) **METHOD FOR THE DIAGNOSIS OF DRY EYE AND BLEPHARITIS**
VERFAHREN FÜR DIE DIAGNOSE VON TROCKENEM AUGE UND BLEPHARITIS
PROCÉDÉ POUR LE DIAGNOSTIC DE L' IL SEC ET DE LA BLÉPHARITE

(30) Priority: 10.08.2010 EP 10382229
(43) Date of publication of application: 19.06.2013
(73) Proprietor: Bioftalmik, S.L., 48160 Derio (Vizcaya) (ES)
(72) Inventor: SUÁREZ CORTÉS, Tatiana, E-48160 Derio (Bizkaia) (ES); SORIA ESPONERA, Javier, E-48160 Derio (Bizkaia) (ES); ACERA OSA, Arantxa, E-48160 Derio (Bizkaia) (ES); GONZALEZ FERNANDEZ, Nerea, E-48160 Derio (Bizkaia) (ES); ECHEVARRÍA ECENARRO, Jaime, E-48903 Baracaldo (Bizkaia) (ES)
(74) Representative: ABG Patentes, S.L.
(86) International application number: PCT/EP2011/063751
(87) International publication number: WO 2012/020046

(56) References cited:
- WO-A1-92/10756
- WO-A2-2007/140976
- US-A1- 2009 258 828
- NICHOLS JASON J ET AL: "Mass spectrometry-based proteomic analyses in contact lens-related dry eye.", CORNEA DEC 2009 LNKD- PUBMED:19770725, vol. 28, no. 10, December 2009 (2009-12), pages 1109-1117, XP008129932, ISSN: 1536-4798
- VERSURA P ET AL: "Proteomic analysis in hyperevaporative dry eye patients", RIVISTA ITALIANA DELLA MEDICINA DI LABORATORIO 2009 SIMEL SOCIETA ITALIANA DI MEDICINA DI LABORATORIO ITA, vol. 5, no. 4, 2009, pages 290-297, XP002612107, ISSN: 1825-859X
- VERSURA P ET AL: "Tear proteomics in evaporative dry eye disease.", EYE (LONDON, ENGLAND) AUG 2010 LNKD- PUBMED:20150925, vol. 24, no. 8, 12 February 2010 (2010-02-12), pages 1396-1402, XP008129926, ISSN: 1476-5454
- KOO BON-SUK ET AL: "Comparative analysis of the tear protein expression in blepharitis patients using two-dimensional electrophoresis", JOURNAL OF PROTEOME RESEARCH, vol. 4, no. 3, May 2005 (2005-05), pages 719-724, XP002631281, ISSN: 1535-3893
- STEINFELD SERGE ET AL: "Big prolactin 60 kDa is overexpressed in salivary glandular epithelial cells from patients with Sjogren's syndrome", LABORATORY INVESTIGATION, vol. 80, no. 2, February 2000 (2000-02), pages 239-247, XP002631283, ISSN: 0023-6837
- DOUGLAS R. FULLEN ET AL: 'Expression of S100A6 protein in a broad spectrum of cutaneous tumors using tissue microarrays' JOURNAL OF CUTANEOUS PATHOLOGY. vol. 35, 01 November 2008, DK, pages 28 - 34, XP055247776 DOI: 10.1111/j.1600-0560.2007.00866.x ISSN: 0303-6987

### SEQUENCE LISTING

<110> Bioftalmik, S.L.
<120> METHOD FOR THE DIAGNOSIS OF DRY EYE AND BLEPHARITIS
<130> P5744PC00
<150> EP10382229.2
   <151> 2010-08-10
<160> 19
<170> PatentIn version 3.5
<210> 1
   <211> 141
   <212> PRT
   <213> Homo sapiens
<400> 1
   Met Ala Arg Pro Leu Cys Thr Leu Leu Leu Leu Met Ala Thr Leu Ala
   1               5                   10                  15
   Gly Ala Leu Ala Ser Ser Ser Lys Glu Glu Asn Arg Ile Ile Pro Gly
               20                  25                  30
   Gly Ile Tyr Asp Ala Asp Leu Asn Asp Glu Trp Val Gln Arg Ala Leu
           35                  40                  45
   His Phe Ala Ile Ser Glu Tyr Asn Lys Ala Thr Glu Asp Glu Tyr Tyr
       50                  55                  60
   Arg Arg Pro Leu Gln Val Leu Arg Ala Arg Glu Gln Thr Phe Gly Gly
   65                  70                  75                  80
   Val Asn Tyr Phe Phe Asp Val Glu Val Gly Arg Thr Ile Cys Thr Lys
                   85                  90                  95
   Ser Gln Pro Asn Leu Asp Thr Cys Ala Phe His Glu Gln Pro Glu Leu
               100                 105                 110
   Gln Lys Lys Gln Leu Cys Ser Phe Glu Ile Tyr Glu Val Pro Trp Glu
           115                 120                 125
   Asp Arg Met Ser Leu Val Asn Ser Arg Cys Gln Glu Ala
       130                 135                 140
<210> 2
   <211> 141
   <212> PRT
   <213> homo sapiens
<400> 2
   Met Ala Gln His Leu Ser Thr Leu Leu Leu Leu Leu Ala Thr Leu Ala
   1               5                   10                  15
   Val Ala Leu Ala Trp Ser Pro Lys Glu Glu Asp Arg Ile Ile Pro Gly
               20                  25                  30
   Gly Ile Tyr Asn Ala Asp Leu Asn Asp Glu Trp Val Gln Arg Ala Leu
           35                  40                  45
   His Phe Ala Ile Ser Glu Tyr Asn Lys Ala Thr Lys Asp Asp Tyr Tyr
       50                  55                  60
   Arg Arg Pro Leu Arg Val Leu Arg Ala Arg Gln Gln Thr Val Gly Gly
   65                  70                  75                  80
   Val Asn Tyr Phe Phe Asp Val Glu Val Gly Arg Thr Ile Cys Thr Lys
                   85                  90                  95
   Ser Gln Pro Asn Leu Asp Thr Cys Ala Phe His Glu Gln Pro Glu Leu
               100                 105                 110
   Gln Lys Lys Gln Leu Cys Ser Phe Glu Ile Tyr Glu Val Pro Trp Glu
           115                 120                 125
   Asn Arg Arg Ser Leu Val Lys Ser Arg Cys Gln Glu Ser
       130                 135                 140
<210> 3
   <211> 90
   <212> PRT
   <213> homo sapiens
<400> 3
   Met Ala Cys Pro Leu Asp Gln Ala Ile Gly Leu Leu Val Ala Ile Phe
   1               5                   10                  15
   His Lys Tyr Ser Gly Arg Glu Gly Asp Lys His Thr Leu Ser Lys Lys
               20                  25                  30
   Glu Leu Lys Glu Leu Ile Gln Lys Glu Leu Thr Ile Gly Ser Lys Leu
           35                  40                  45
   Gln Asp Ala Glu Ile Ala Arg Leu Met Glu Asp Leu Asp Arg Asn Lys
       50                  55                  60
   Asp Gln Glu Val Asn Phe Gln Glu Tyr Val Thr Phe Leu Gly Ala Leu
   65                  70                  75                  80
   Ala Leu Ile Tyr Asn Glu Ala Leu Lys Gly
                   85                  90
<210> 4
   <211> 114
   <212> PRT
   <213> homo sapiens
<400> 4
   Met Thr Cys Lys Met Ser Gln Leu Glu Arg Asn Ile Glu Thr Ile Ile
   1               5                   10                  15
   Asn Thr Phe His Gln Tyr Ser Val Lys Leu Gly His Pro Asp Thr Leu
               20                  25                  30
   Asn Gln Gly Glu Phe Lys Glu Leu Val Arg Lys Asp Leu Gln Asn Phe
           35                  40                  45
   Leu Lys Lys Glu Asn Lys Asn Glu Lys Val Ile Glu His Ile Met Glu
       50                  55                  60
   Asp Leu Asp Thr Asn Ala Asp Lys Gln Leu Ser Phe Glu Glu Phe Ile
   65                  70                  75                  80
   Met Leu Met Ala Arg Leu Thr Trp Ala Ser His Glu Lys Met His Glu
                   85                  90                  95
   Gly Asp Glu Gly Pro Gly His His His Lys Pro Gly Leu Gly Glu Gly
               100                 105                 110
   Thr Pro
<210> 5
   <211> 210
   <212> PRT
   <213> homo sapiens
<400> 5
   Met Pro Pro Tyr Thr Val Val Tyr Phe Pro Val Arg Gly Arg Cys Ala
   1               5                   10                  15
   Ala Leu Arg Met Leu Leu Ala Asp Gln Gly Gln Ser Trp Lys Glu Glu
               20                  25                  30
   Val Val Thr Val Glu Thr Trp Gln Glu Gly Ser Leu Lys Ala Ser Cys
           35                  40                  45
   Leu Tyr Gly Gln Leu Pro Lys Phe Gln Asp Gly Asp Leu Thr Leu Tyr
       50                  55                  60
   Gln Ser Asn Thr Ile Leu Arg His Leu Gly Arg Thr Leu Gly Leu Tyr
   65                  70                  75                  80
   Gly Lys Asp Gln Gln Glu Ala Ala Leu Val Asp Met Val Asn Asp Gly
                   85                  90                  95
   Val Glu Asp Leu Arg Cys Lys Tyr Ile Ser Leu Ile Tyr Thr Asn Tyr
               100                 105                 110
   Glu Ala Gly Lys Asp Asp Tyr Val Lys Ala Leu Pro Gly Gln Leu Lys
           115                 120                 125
   Pro Phe Glu Thr Leu Leu Ser Gln Asn Gln Gly Gly Lys Thr Phe Ile
       130                 135                 140
   Val Gly Asp Gln Ile Ser Phe Ala Asp Tyr Asn Leu Leu Asp Leu Leu
   145                 150                 155                 160
   Leu Ile His Glu Val Leu Ala Pro Gly Cys Leu Asp Ala Phe Pro Leu
                   165                 170                 175
   Leu Ser Ala Tyr Val Gly Arg Leu Ser Ala Arg Pro Lys Leu Lys Ala
               180                 185                 190
   Phe Leu Ala Ser Pro Glu Tyr Val Asn Leu Pro Ile Asn Gly Asn Gly
           195                 200                 205
   Lys Gln
       210
<210> 6
   <211> 136
   <212> PRT
   <213> homo sapiens
<400> 6
   Met Ser Asn Val Pro His Lys Ser Ser Leu Pro Glu Gly Ile Arg Pro
   1               5                   10                  15
   Gly Thr Val Leu Arg Ile Arg Gly Leu Val Pro Pro Asn Ala Ser Arg
               20                  25                  30
   Phe His Val Asn Leu Leu Cys Gly Glu Glu Gln Gly Ser Asp Ala Ala
           35                  40                  45
   Leu His Phe Asn Pro Arg Leu Asp Thr Ser Glu Val Val Phe Asn Ser
       50                  55                  60
   Lys Glu Gln Gly Ser Trp Gly Arg Glu Glu Arg Gly Pro Gly Val Pro
   65                  70                  75                  80
   Phe Gln Arg Gly Gln Pro Phe Glu Val Leu Ile Ile Ala Ser Asp Asp
                   85                  90                  95
   Gly Phe Lys Ala Val Val Gly Asp Ala Gln Tyr His His Phe Arg His
               100                 105                 110
   Arg Leu Pro Leu Ala Arg Val Arg Leu Val Glu Val Gly Gly Asp Val
           115                 120                 125
   Gln Leu Asp Ser Val Arg Ile Phe
       130                 135
<210> 7
   <211> 346
   <212> PRT
   <213> homo sapiens
<400> 7
   Met Ala Met Val Ser Glu Phe Leu Lys Gln Ala Trp Phe Ile Glu Asn
   1               5                   10                  15
   Glu Glu Gln Glu Tyr Val Gln Thr Val Lys Ser Ser Lys Gly Gly Pro
               20                  25                  30
   Gly Ser Ala Val Ser Pro Tyr Pro Thr Phe Asn Pro Ser Ser Asp Val
           35                  40                  45
   Ala Ala Leu His Lys Ala Ile Met Val Lys Gly Val Asp Glu Ala Thr
       50                  55                  60
   Ile Ile Asp Ile Leu Thr Lys Arg Asn Asn Ala Gln Arg Gln Gln Ile
   65                  70                  75                  80
   Lys Ala Ala Tyr Leu Gln Glu Thr Gly Lys Pro Leu Asp Glu Thr Leu
                   85                  90                  95
   Lys Lys Ala Leu Thr Gly His Leu Glu Glu Val Val Leu Ala Leu Leu
               100                 105                 110
   Lys Thr Pro Ala Gln Phe Asp Ala Asp Glu Leu Arg Ala Ala Met Lys
           115                 120                 125
   Gly Leu Gly Thr Asp Glu Asp Thr Leu Ile Glu Ile Leu Ala Ser Arg
       130                 135                 140
   Thr Asn Lys Glu Ile Arg Asp Ile Asn Arg Val Tyr Arg Glu Glu Leu
   145                 150                 155                 160
   Lys Arg Asp Leu Ala Lys Asp Ile Thr Ser Asp Thr Ser Gly Asp Phe
                   165                 170                 175
   Arg Asn Ala Leu Leu Ser Leu Ala Lys Gly Asp Arg Ser Glu Asp Phe
               180                 185                 190
   Gly Val Asn Glu Asp Leu Ala Asp Ser Asp Ala Arg Ala Leu Tyr Glu
           195                 200                 205
   Ala Gly Glu Arg Arg Lys Gly Thr Asp Val Asn Val Phe Asn Thr Ile
       210                 215                 220
   Leu Thr Thr Arg Ser Tyr Pro Gln Leu Arg Arg Val Phe Gln Lys Tyr
   225                 230                 235                 240
   Thr Lys Tyr Ser Lys His Asp Met Asn Lys Val Leu Asp Leu Glu Leu
                   245                 250                 255
   Lys Gly Asp Ile Glu Lys Cys Leu Thr Ala Ile Val Lys Cys Ala Thr
               260                 265                 270
   Ser Lys Pro Ala Phe Phe Ala Glu Lys Leu His Gln Ala Met Lys Gly
           275                 280                 285
   Val Gly Thr Arg His Lys Ala Leu Ile Arg Ile Met Val Ser Arg Ser
       290                 295                 300
   Glu Ile Asp Met Asn Asp Ile Lys Ala Phe Tyr Gln Lys Met Tyr Gly
   305                 310                 315                 320
   Ile Ser Leu Cys Gln Ala Ile Leu Asp Glu Thr Lys Gly Asp Tyr Glu
                   325                 330                 335
   Lys Ile Leu Val Ala Leu Cys Gly Gly Asn
               340                 345
<210> 8
   <211> 391
   <212> PRT
   <213> homo sapiens
<400> 8
   Met Asp Ser Leu Gly Ala Val Ser Thr Arg Leu Gly Phe Asp Leu Phe
   1               5                   10                  15
   Lys Glu Leu Lys Lys Thr Asn Asp Gly Asn Ile Phe Phe Ser Pro Val
               20                  25                  30
   Gly Ile Leu Thr Ala Ile Gly Met Val Leu Leu Gly Thr Arg Gly Ala
           35                  40                  45
   Thr Ala Ser Gln Leu Glu Glu Val Phe His Ser Glu Lys Glu Thr Lys
       50                  55                  60
   Ser Ser Arg Ile Lys Ala Glu Glu Lys Glu Val Ile Glu Asn Thr Glu
   65                  70                  75                  80
   Ala Val His Gln Gln Phe Gln Lys Phe Leu Thr Glu Ile Ser Lys Leu
                   85                  90                  95
   Thr Asn Asp Tyr Glu Leu Asn Ile Thr Asn Arg Leu Phe Gly Glu Lys
               100                 105                 110
   Thr Tyr Leu Phe Leu Gln Lys Tyr Leu Asp Tyr Val Glu Lys Tyr Tyr
           115                 120                 125
   His Ala Ser Leu Glu Pro Val Asp Phe Val Asn Ala Ala Asp Glu Ser
       130                 135                 140
   Arg Lys Lys Ile Asn Ser Trp Val Glu Ser Lys Thr Asn Glu Lys Ile
   145                 150                 155                 160
   Lys Asp Leu Phe Pro Asp Gly Ser Ile Ser Ser Ser Thr Lys Leu Val
                   165                 170                 175
   Leu Val Asn Met Val Tyr Phe Lys Gly Gln Trp Asp Arg Glu Phe Lys
               180                 185                 190
   Lys Glu Asn Thr Lys Glu Glu Lys Phe Trp Met Asn Lys Ser Thr Ser
           195                 200                 205
   Lys Ser Val Gln Met Met Thr Gln Ser His Ser Phe Ser Phe Thr Phe
       210                 215                 220
   Leu Glu Asp Leu Gln Ala Lys Ile Leu Gly Ile Pro Tyr Lys Asn Asn
   225                 230                 235                 240
   Asp Leu Ser Met Phe Val Leu Leu Pro Asn Asp Ile Asp Gly Leu Glu
                   245                 250                 255
   Lys Ile Ile Asp Lys Ile Ser Pro Glu Lys Leu Val Glu Trp Thr Ser
   260 265 270
   Pro Gly His Met Glu Glu Arg Lys Val Asn Leu His Leu Pro Arg Phe
   275 280 285
   Glu Val Glu Asp Gly Tyr Asp Leu Glu Ala Val Leu Ala Ala Met Gly
   290 295 300
   Met Gly Asp Ala Phe Ser Glu His Lys Ala Asp Tyr Ser Gly Met Ser
   305 310 315 320
   Ser Gly Ser Gly Leu Tyr Ala Gln Lys Phe Leu His Ser Ser Phe Val
   325 330 335
   Ala Val Thr Glu Glu Gly Thr Glu Ala Ala Ala Ala Thr Gly Ile Gly
   340 345 350
   Phe Thr Val Thr Ser Ala Pro Gly His Glu Asn Val His Cys Asn His
   355 360 365
   Pro Phe Leu Phe Phe Ile Arg His Asn Glu Ser Asn Ser Ile Leu Phe
   370 375 380
   Phe Gly Arg Phe Ser Ser Pro
   385 390
<210> 9
   <211> 331
   <212> PRT
   <213> homo sapiens
<400> 9
   Met Ala Arg Gly Gly Arg Gly Arg Arg Leu Gly Leu Ala Leu Gly Leu
   1 5 10 15
   Leu Leu Ala Leu Val Leu Ala Pro Arg Val Leu Arg Ala Lys Pro Thr
   20 25 30
   Val Arg Lys Glu Arg Val Val Arg Pro Asp Ser Glu Leu Gly Glu Arg
   35 40 45
   Pro Pro Glu Asp Asn Gln Ser Phe Gln Tyr Asp His Glu Ala Phe Leu
   50 55 60
   Gly Lys Glu Asp Ser Lys Thr Phe Asp Gln Leu Thr Pro Asp Glu Ser
   65 70 75 80
   Lys Glu Arg Leu Gly Lys Ile Val Asp Arg Ile Asp Asn Asp Gly Asp
                   85                  90                  95
   Gly Phe Val Thr Thr Glu Glu Leu Lys Thr Trp Ile Lys Arg Val Gln
               100                 105                 110
   Lys Arg Tyr Ile Phe Asp Asn Val Ala Lys Val Trp Lys Asp Tyr Asp
           115                 120                 125
   Arg Asp Lys Asp Asp Lys Ile Ser Trp Glu Glu Tyr Lys Gln Ala Thr
       130                 135                 140
   Tyr Gly Tyr Tyr Leu Gly Asn Pro Ala Glu Phe His Asp Ser Ser Asp
   145                 150                 155                 160
   His His Thr Phe Lys Lys Met Leu Pro Arg Asp Glu Arg Arg Phe Lys
                   165                 170                 175
   Ala Ala Asp Leu Asn Gly Asp Leu Thr Ala Thr Arg Glu Glu Phe Thr
               180                 185                 190
   Ala Phe Leu His Pro Glu Glu Phe Glu His Met Lys Glu Ile Val Val
           195                 200                 205
   Leu Glu Thr Leu Glu Asp Ile Asp Lys Asn Gly Asp Gly Phe Val Asp
       210                 215                 220
   Gln Asp Glu Tyr Ile Ala Asp Met Phe Ser His Glu Glu Asn Gly Pro
   225                 230                 235                 240
   Glu Pro Asp Trp Val Leu Ser Glu Arg Glu Gln Phe Asn Glu Phe Arg
                   245                 250                 255
   Asp Leu Asn Lys Asp Gly Lys Leu Asp Lys Asp Glu Ile Arg His Trp
               260                 265                 270
   Ile Leu Pro Gln Asp Tyr Asp His Ala Gln Ala Glu Ala Arg His Leu
           275                 280                 285
   Val Tyr Glu Ser Asp Lys Asn Lys Asp Glu Lys Leu Thr Lys Glu Glu
       290                 295                 300
   Ile Leu Glu Asn Trp Asn Met Phe Val Gly Ser Gln Ala Thr Asn Tyr
   305                 310                 315                 320
   Gly Glu Asp Leu Thr Lys Asn His Asp Glu Leu
   325 330
<210> 10
   <211> 222
   <212> PRT
   <213> homo sapiens
<400> 10
<210> 11
   <211> 425
   <212> PRT
   <213> homo sapiens
<400> 11
<210> 12
   <211> 427
   <212> PRT
   <213> homo sapiens
<400> 12
   Met Ser Arg Ser Lys Arg Asp Asn Asn Phe Tyr Ser Val Glu Ile Gly
   1               5                   10                  15
   Asp Ser Thr Phe Thr Val Leu Lys Arg Tyr Gln Asn Leu Lys Pro Ile
               20                  25                  30
   Gly Ser Gly Ala Gln Gly Ile Val Cys Ala Ala Tyr Asp Ala Ile Leu
           35                  40                  45
   Glu Arg Asn Val Ala Ile Lys Lys Leu Ser Arg Pro Phe Gln Asn Gln
       50                  55                  60
   Thr His Ala Lys Arg Ala Tyr Arg Glu Leu Val Leu Met Lys Cys Val
   65                  70                  75                  80
   Asn His Lys Asn Ile Ile Gly Leu Leu Asn Val Phe Thr Pro Gln Lys
                   85                  90                  95
   Ser Leu Glu Glu Phe Gln Asp Val Tyr Ile Val Met Glu Leu Met Asp
               100                 105                 110
   Ala Asn Leu Cys Gln Val Ile Gln Met Glu Leu Asp His Glu Arg Met
           115                 120                 125
   Ser Tyr Leu Leu Tyr Gln Met Leu Cys Gly Ile Lys His Leu His Ser
       130                 135                 140
   Ala Gly Ile Ile His Arg Asp Leu Lys Pro Ser Asn Ile Val Val Lys
   145                 150                 155                 160
   Ser Asp Cys Thr Leu Lys Ile Leu Asp Phe Gly Leu Ala Arg Thr Ala
                   165                 170                 175
   Gly Thr Ser Phe Met Met Thr Pro Tyr Val Val Thr Arg Tyr Tyr Arg
               180                 185                 190
   Ala Pro Glu Val Ile Leu Gly Met Gly Tyr Lys Glu Asn Val Asp Leu
           195                 200                 205
   Trp Ser Val Gly Cys Ile Met Gly Glu Met Val Cys His Lys Ile Leu
       210                 215                 220
   Phe Pro Gly Arg Asp Tyr Ile Asp Gln Trp Asn Lys Val Ile Glu Gln
   225                 230                 235                 240
   Leu Gly Thr Pro Cys Pro Glu Phe Met Lys Lys Leu Gln Pro Thr Val
                   245                 250                 255
   Arg Thr Tyr Val Glu Asn Arg Pro Lys Tyr Ala Gly Tyr Ser Phe Glu
                   260                 265                 270
   Lys Leu Phe Pro Asp Val Leu Phe Pro Ala Asp Ser Glu His Asn Lys
               275                 280                 285
   Leu Lys Ala Ser Gln Ala Arg Asp Leu Leu Ser Lys Met Leu Val Ile
       290                 295                 300
   Asp Ala Ser Lys Arg Ile Ser Val Asp Glu Ala Leu Gln His Pro Tyr
   305                 310                 315                 320
   Ile Asn Val Trp Tyr Asp Pro Ser Glu Ala Glu Ala Pro Pro Pro Lys
                   325                 330                 335
   Ile Pro Asp Lys Gln Leu Asp Glu Arg Glu His Thr Ile Glu Glu Trp
               340                 345                 350
   Lys Glu Leu Ile Tyr Lys Glu Val Met Asp Leu Glu Glu Arg Thr Lys
           355                 360                 365
   Asn Gly Val Ile Arg Gly Gln Pro Ser Pro Leu Gly Ala Ala Val Ile
       370                 375                 380
   Asn Gly Ser Gln His Pro Ser Ser Ser Ser Ser Val Asn Asp Val Ser
   385                 390                 395                 400
   Ser Met Ser Thr Asp Pro Thr Leu Ala Ser Asp Thr Asp Ser Ser Leu
                   405                 410                 415
   Glu Ala Ala Ala Gly Pro Leu Gly Cys Cys Arg
               420                 425
<210> 13
   <211> 795
   <212> PRT
   <213> homo sapiens
<400> 13
<210> 14
   <211> 218
   <212> PRT
   <213> homo sapiens
<400> 14
   Met Pro Met Thr Leu Gly Tyr Trp Asn Ile Arg Gly Leu Ala His Ser
   1               5                   10                  15
   Ile Arg Leu Leu Leu Glu Tyr Thr Asp Ser Ser Tyr Glu Glu Lys Lys
               20                  25                  30
   Tyr Thr Met Gly Asp Ala Pro Asp Tyr Asp Arg Ser Gln Trp Leu Asn
           35                  40                  45
   Glu Lys Phe Lys Leu Gly Leu Asp Phe Pro Asn Leu Pro Tyr Leu Ile
       50                  55                  60
   Asp Gly Thr His Lys Ile Thr Gln Ser Asn Ala Ile Leu Arg Tyr Ile
   65                  70                  75                  80
   Ala Arg Lys His Asn Leu Cys Gly Glu Ser Glu Lys Glu Gln Ile Arg
                   85                  90                  95
   Glu Asp Ile Leu Glu Asn Gln Phe Met Asp Ser Arg Met Gln Leu Ala
               100                 105                 110
   Lys Leu Cys Tyr Asp Pro Asp Phe Glu Lys Leu Lys Pro Glu Tyr Leu
           115                 120                 125
   Gln Ala Leu Pro Glu Met Leu Lys Leu Tyr Ser Gln Phe Leu Gly Lys
       130                 135                 140
   Gln Pro Trp Phe Leu Gly Asp Lys Ile Thr Phe Val Asp Phe Ile Ala
   145                 150                 155                 160
   Tyr Asp Val Leu Glu Arg Asn Gln Val Phe Glu Pro Ser Cys Leu Asp
                   165                 170                 175
   Ala Phe Pro Asn Leu Lys Asp Phe Ile Ser Arg Phe Glu Gly Leu Glu
               180                 185                 190
   Lys Ile Ser Ala Tyr Met Lys Ser Ser Arg Phe Leu Pro Arg Pro Val
           195                 200                 205
   Phe Thr Lys Met Ala Val Trp Gly Asn Lys
       210                 215
<210> 15
   <211> 222
   <212> PRT
   <213> homo sapiens
<400> 15
   Met Ala Ala Arg Pro Lys Leu His Tyr Pro Asn Gly Arg Gly Arg Met
   1               5                   10                  15
   Glu Ser Val Arg Trp Val Leu Ala Ala Ala Gly Val Glu Phe Asp Glu
               20                  25                  30
   Glu Phe Leu Glu Thr Lys Glu Gln Leu Tyr Lys Leu Gln Asp Gly Asn
           35                  40                  45
   His Leu Leu Phe Gln Gln Val Pro Met Val Glu Ile Asp Gly Met Lys
       50                  55                  60
   Leu Val Gln Thr Arg Ser Ile Leu His Tyr Ile Ala Asp Lys His Asn
   65                  70                  75                  80
   Leu Phe Gly Lys Asn Leu Lys Glu Arg Thr Leu Ile Asp Met Tyr Val
                   85                  90                  95
   Glu Gly Thr Leu Asp Leu Leu Glu Leu Leu Ile Met His Pro Phe Leu
               100                 105                 110
   Lys Pro Asp Asp Gln Gln Lys Glu Val Val Asn Met Ala Gln Lys Ala
           115                 120                 125
   Ile Ile Arg Tyr Phe Pro Val Phe Glu Lys Ile Leu Arg Gly His Gly
       130                 135                 140
   Gln Ser Phe Leu Val Gly Asn Gln Leu Ser Leu Ala Asp Val Ile Leu
   145                 150                 155                 160
   Leu Gln Thr Ile Leu Ala Leu Glu Glu Lys Ile Pro Asn Ile Leu Ser
                   165                 170                 175
   Ala Phe Pro Phe Leu Gln Glu Tyr Thr Val Lys Leu Ser Asn Ile Pro
               180                 185                 190
   Thr Ile Lys Arg Phe Leu Glu Pro Gly Ser Lys Lys Lys Pro Pro Pro
           195                 200                 205
   Asp Glu Ile Tyr Val Arg Thr Val Tyr Asn Ile Phe Arg Pro
       210                 215                 220
<210> 16
   <211> 408
   <212> PRT
   <213> homo sapiens
<400> 16
   Met Ala Glu Asn Gly Asp Asn Glu Lys Met Ala Ala Leu Glu Ala Lys
   1               5                   10                  15
   Ile Cys His Gln Ile Glu Tyr Tyr Phe Gly Asp Phe Asn Leu Pro Arg
               20                  25                  30
   Asp Lys Phe Leu Lys Glu Gln Ile Lys Leu Asp Glu Gly Trp Val Pro
           35                  40                  45
   Leu Glu Ile Met Ile Lys Phe Asn Arg Leu Asn Arg Leu Thr Thr Asp
       50                  55                  60
   Phe Asn Val Ile Val Glu Ala Leu Ser Lys Ser Lys Ala Glu Leu Met
   65                  70                  75                  80
   Glu Ile Ser Glu Asp Lys Thr Lys Ile Arg Arg Ser Pro Ser Lys Pro
                   85                  90                  95
   Leu Pro Glu Val Thr Asp Glu Tyr Lys Asn Asp Val Lys Asn Arg Ser
               100                 105                 110
   Val Tyr Ile Lys Gly Phe Pro Thr Asp Ala Thr Leu Asp Asp Ile Lys
           115                 120                 125
   Glu Trp Leu Glu Asp Lys Gly Gln Val Leu Asn Ile Gln Met Arg Arg
       130                 135                 140
   Thr Leu His Lys Ala Phe Lys Gly Ser Ile Phe Val Val Phe Asp Ser
   145                 150                 155                 160
   Ile Glu Ser Ala Lys Lys Phe Val Glu Thr Pro Gly Gln Lys Tyr Lys
                   165                 170                 175
   Glu Thr Asp Leu Leu Ile Leu Phe Lys Asp Asp Tyr Phe Ala Lys Lys
               180                 185                 190
   Asn Glu Glu Arg Lys Gln Asn Lys Val Glu Ala Lys Leu Arg Ala Lys
           195                 200                 205
   Gln Glu Gln Glu Ala Lys Gln Lys Leu Glu Glu Asp Ala Glu Met Lys
       210                 215                 220
   Ser Leu Glu Glu Lys Ile Gly Cys Leu Leu Lys Phe Ser Gly Asp Leu
   225                 230                 235                 240
   Asp Asp Gln Thr Cys Arg Glu Asp Leu His Ile Leu Phe Ser Asn His
                   245                 250                 255
   Gly Glu Ile Lys Trp Ile Asp Phe Val Arg Gly Ala Lys Glu Gly Ile
               260                 265                 270
   Ile Leu Phe Lys Glu Lys Ala Lys Glu Ala Leu Gly Lys Ala Lys Asp
           275                 280                 285
   Ala Asn Asn Gly Asn Leu Gln Leu Arg Asn Lys Glu Val Thr Trp Glu
       290                 295                 300
   Val Leu Glu Gly Glu Val Glu Lys Glu Ala Leu Lys Lys Ile Ile Glu
   305                 310                 315                 320
   Asp Gln Gln Glu Ser Leu Asn Lys Trp Lys Ser Lys Gly Arg Arg Phe
                   325                 330                 335
   Lys Gly Lys Gly Lys Gly Asn Lys Ala Ala Gln Pro Gly Ser Gly Lys
               340                 345                 350
   Gly Lys Val Gln Phe Gln Gly Lys Lys Thr Lys Phe Ala Ser Asp Asp
           355                 360                 365
   Glu His Asp Glu His Asp Glu Asn Gly Ala Thr Gly Pro Val Lys Arg
       370                 375                 380
   Ala Arg Glu Glu Thr Asp Lys Glu Glu Pro Ala Ser Lys Gln Gln Lys
   385                 390                 395                 400
   Thr Glu Asn Gly Ala Gly Asp Gln
                   405
<210> 17
   <211> 505
   <212> PRT
   <213> homo sapiens
<400> 17
<210> 18
   <211> 90
   <212> PRT
   <213> homo sapiens
<400> 18
   Met Arg Leu Ser Val Cys Leu Leu Leu Leu Thr Leu Ala Leu Cys Cys
   1               5                   10                  15
   Tyr Arg Ala Asn Ala Val Val Cys Gln Ala Leu Gly Ser Glu Ile Thr
               20                  25                  30
   Gly Phe Leu Leu Ala Gly Lys Pro Val Phe Lys Phe Gln Leu Ala Lys
           35                  40                  45
   Phe Lys Ala Pro Leu Glu Ala Val Ala Ala Lys Met Glu Val Lys Lys
       50                  55                  60
   Cys Val Asp Thr Met Ala Tyr Glu Lys Arg Val Leu Ile Thr Lys Thr
   65                  70                  75                  80
   Leu Gly Lys Ile Ala Glu Lys Cys Asp Arg
                   85                  90
<210> 19
   <211> 214
   <212> PRT
   <213> homo sapiens
<400> 19
   Met Gly Leu Ala Gly Val Cys Ala Leu Arg Arg Ser Ala Gly Tyr Ile
   1               5                   10                  15
   Leu Val Gly Gly Ala Gly Gly Gln Ser Ala Ala Ala Ala Ala Arg Arg
               20                  25                  30
   Cys Ser Glu Gly Glu Trp Ala Ser Gly Gly Val Arg Ser Phe Ser Arg
           35                  40                  45
   Ala Ala Ala Ala Met Ala Pro Ile Lys Val Gly Asp Ala Ile Pro Ala
       50                  55                  60
   Val Glu Val Phe Glu Gly Glu Pro Gly Asn Lys Val Asn Leu Ala Glu
   65                  70                  75                  80
   Leu Phe Lys Gly Lys Lys Gly Val Leu Phe Gly Val Pro Gly Ala Phe
                   85                  90                  95
   Thr Pro Gly Cys Ser Lys Thr His Leu Pro Gly Phe Val Glu Gln Ala
               100                 105                 110
   Glu Ala Leu Lys Ala Lys Gly Val Gln Val Val Ala Cys Leu Ser Val
           115                 120                 125
   Asn Asp Ala Phe Val Thr Gly Glu Trp Gly Arg Ala His Lys Ala Glu
       130                 135                 140
   Gly Lys Val Arg Leu Leu Ala Asp Pro Thr Gly Ala Phe Gly Lys Glu
   145                 150                 155                 160
   Thr Asp Leu Leu Leu Asp Asp Ser Leu Val Ser Ile Phe Gly Asn Arg
                   165                 170                 175
   Arg Leu Lys Arg Phe Ser Met Val Val Gln Asp Gly Ile Val Lys Ala
               180                 185                 190
   Leu Asn Val Glu Pro Asp Gly Thr Gly Leu Thr Cys Ser Leu Ala Pro
           195                 200                 205
   Asn Ile Ile Ser Gln Leu
       210

## Description

### Field of the Invention

The invention is comprised within the field of the disease diagnosis area; specifically, in the development of a method for the diagnosis of dry eye and/or blepharitis in a subject, based on the detection of determined markers.

### Background of the Invention

The dry eye syndrome (also known as keratitis sicca) is an ocular surface disease which may be due to a reduction of the activity of the lacrimal glands with the consequent lower tear production, in which case it is referred to as "hyposecretory dry eye" or to an excessive loss of water of the exposed ocular surface in the presence of a normal secretory function, in which case it is known as "evaporative dry eye".

It is currently defined as a multifactorial disease resulting in ocular discomfort, visual alteration and instability of the tear film, with potential damages of the ocular surface. It is accompanied by an increased osmolarity of the tear film and by inflammation of the ocular surface (DEWS, The Ocular Surface, April 2007, Vol. 5, 2: 69-202).

There are multiple causes which can cause dry eye, being more common in the elderly. It can be caused, among other factors, by the aging process, use of contact lenses, hormonal changes in women, environmental factors, side effects of diseases/medicinal products, laser surgeries for vision correction, destabilization of the tear composition and other chronic eye diseases. The diseases causing dry eyes include vitamin A deficiency, Sjögren's syndrome, rheumatoid arthritis and other rheumatologic diseases, it can also occur due to chemical or thermal burns, and drugs such as atenolol, chlorpheniramine, hydrochlorothiazide, isotretinoin, ketorolac, ketotifen, levocabastine, levofloxacin, oxybutynin or tolterodine. As the disease progresses, there is a thickening of the cornea and a reduction of visual acuity. Other symptoms of dry eyes are a stinging or burning sensation in the eye, foreign body sensation, itching or pruritus, rheum and conjunctival reddening.

Blepharitis is a term which is used to describe the inflammation of the tissue forming the eyelid. Its origin is often due to a malfunctioning of the glands which are located in the eyelid margin. Under normal conditions, these glands produce an oily secretion which aids in lubricating the surface of the eye and the inner side of the eyelids, preventing the evaporation of tears. In subjects with blepharitis, these glands are obstructed, their secretions are stagnant and fatty acids are formed which irritate the ocular surface. The margin of the eyelids is inflamed and reddened in these cases. The eye is irritated and produces secretion of mucus and proteins, and the latter accumulate in the eyelid margin, often creating a crust. The accumulation of these materials provides the optimal conditions for the growth of bacteria, which in turn release toxins which contribute to irritating the eyelids even more and to further aggravating the pathological process. Therefore, in blepharitis there is a chain of events including eyelid gland dysfunction, irritation and formation of small crusts in the eyelid margin, in addition to bacterial infection. The severity of blepharitis varies considerably among individuals. In some cases, it only represents a moderate discomfort, creating a slight, intermittent irritation. In others, it is a more serious disease which can affect vision.

Blepharitis is a common process affecting 5% of the population, with a chronic nature and which is presented in outbreaks. This disease occurs both in men and in women without distinction, but it has a slightly greater incidence in men. Nevertheless, associated with other diseases, it can have an incidence of up to 15% as in the case of ocular cicatricial pemphigoid or of up to 19% in the case of its association with dry eyes. There are two types of blepharitis: anterior (seborrheic or staphylococcal) blepharitis and posterior (hypersecretory or obstructive) blepharitis, the latter may occur due to a meibomian gland dysfunction (MGD). In seborrheic anterior blepharitis, the appearance of the edge of the eyelid is very "oily", with soft and "sticky" plaques or flakes, with abundant yellowish-white secretion at the exit of the glands, with the eyelashes adhered to one another by the oil. Favored by this alteration of glandular secretion, there is bacterial colonization, the most important bacterium causing the infection being a staphylococcus. There is greater reddening due to the direct irritation of the bacteria and their toxins. In staphylococcal posterior blepharitis, there are no oily and sticky flakes as in seborrheic blepharitis, but rather dry, larger crusts which are visible without a microscope. Genuine collarettes are often formed the eyelashes, and it seems like "dandruff" in the eyelashes, with reddened skin and irritated eyes. Posterior blepharitis may occur when the meibomian glands are affected between others, the inner part of the eyelashes which is in contact with the eye also being affected. This blepharitis has other names, such as meibomitis, meibomian gland dysfunction (MGD), meibomian foam, etc., and although the symptoms do not seem to be as significant as seborrheic anterior blepharitis, it is easier for the ocular surface to be altered. Due to the fact that the meibomian glands are in charge of producing the lipid component of tears, if meibomitis occurs, this component is altered and the tears are "of a poor quality", they break and do not remain homogeneously distributed over the surface of the eye. Thus, when blepharitis is referred to as the cause of dry eye, reference is almost always made to posterior blepharitis (a meibomian gland problem). Likewise, anterior and posterior blepharitis can occur simultaneously, in fact it is quite usual. For example, in the case of seborrheic blepharitis, there is an alteration of the glands both in the front part (eyelashes "covered" in oil) and in the rear part (meibomitis).

The diagnosis of dry eye is normally performed with Schirmer's test, which consists of placing a strip of blotting paper hanging from the lower eyelid, keeping the eyes closed for 5 minutes and observing what length of the paper is wet with tears, the normal length being 15 mm. This test has the drawback that tears can be generated due to the irritation with the paper and it is often recommended to use local anesthesia. Therefore, several new tests have been developed over time, such as the measurement of lactoferrin, since it seems that the amount of this molecule is closely related to tear production. Patients with low tear production and dry eyes have low levels of lactoferrin. There is also another method which consists of measuring the lysozyme concentration in tears. Another test consists of adding drops of fluorescein in the eye of the patient, such that the fluorescein should pass from the tear duct to the nose in 2 minutes. If the patient does not have enough tears to entrain the marker, this time will be longer.

In relation to the diagnosis of blepharitis, an evaluation of the eyelids during an eye examination is generally enough to diagnose said disease. Another possible way to diagnose blepharitis consists of analyzing a sample from the eye of the patient and checking if it has any bacterial infection and even possible allergies which may be causing blepharitis.

Both diseases, dry eye syndrome and blepharitis, are closely related and often occur together in the patient (being referred to as mixed dry eyes-blepharitis pathology). It is known that approximately 70% of the patients with dry eyes have associated blepharitis (Lemp MA. Report of the National Eye Institute/Industry workshop on Clinical Trials in Dry Eyes. CLAO J. 1995; 21:221-32). It is therefore important to differentiate if the patient suffers only from dry eye syndrome, blepharitis or both pathologies.

US patent application US2009/258828 describes a method for the diagnosis of dry eye by determining the levels of the S100 proteins S100 A9, S 100 A1 and S100 A11.

Nichols et al. (Cornea Dec., 2009, 28: 1109-1117) describe a proteomic method for the identification of protein biomarkers associated with dry eye in contact lens wearers as well as the identification of different polypeptides, the expression levels of which are either increased or decreased in the dry eye state. Versura et al. (Rivista Italiana della Medicina di Laboratorio, 2009, 5:290-297) describe a proteomic analysis in hyperevaporative dry eye patients and the identification of different polypeptides the expression levels of which are altered in this type of patients.

Versura et al. (Eye, 2010, 24: 1396-1402) describes a proteomic analysis in hyperevaporative dry eye patients and the identification of different polypeptides the expression levels of which are altered in this type of patients.

Koo et al. (J.Proteome Res., 2005, 24: 719-724) describes a proteomic analisys of the tears in patients suffering blepharitis and the identification of proteins, the expression levels of which are altered in blepharitis patients. International patent application WO2007140976 describes a method for assessing Alzheimer's Disease in vitro comprising measuring in a body fluid sample the level of Peroxiredoxin 5 or Microsomal glutathione S-transferase 3 (MGST-3).

International patent application WO92/10756 describes methods for detecting elevated levels of phospholipase A2, activating protein in persons suspected of having rheumatoid arthritis to thereby indicate the presence of rheumathoid arthritis. Steinfeld et al. (Lab. Invest., 2000, 80: 239-247) describes that the expression of big prolactin 60 kDa is overexpressed in salivary glandular epithelial cells from patients with Sjögren's Syndrome

It is therefore necessary to develop methods for the diagnosis of dry eye and blepharitis and a method with which both syndromes can be differentiated and which overcomes the mentioned drawbacks; it would be particularly desirable for said method to be simple and sufficiently sensitive for differentiating between both pathologies.

### Summary of the Invention

The authors of the present invention have discovered a new group of -molecular markers which allow the detection and/or the diagnosis of dry eye and blepharitis and, furthermore, allow discriminating between patients suffering from dry eye and blepharitis.

In one aspect, the invention relates to an *in vitro* method for diagnosing dry eye in a subject which comprises:
(i) determining the level of S100A6 protein in a tear sample from said subject, and
(ii) comparing the level of said protein with the level of said protein in a reference sample,
wherein if the subject has dry eye, the level of S100A6 protein is elevated.

In another aspect, the invention relates to a kit comprising at least one reagent for the detection of protein S100A6 for its use in the *in vitro* method for diagnosing dry eye in a subject according to claim 1, said kit additionally comprising reagents for the detection of one or more proteins selected from the group of Serpin B13, Reticulocalbin 1, Superoxide dismutase, SMAD3, GSTM2, GSTA4 and SSB.

Which comprises:
(i) determining the level of one or more proteins selected from the group of Lipophilin A, Peroxiredoxin 5, Cystatin S, PLAA, S100A4, S100A6, GSTP1, Annexin 1, Annexin 11, JNK and Galectin 7 in a sample from a subject; and
(ii) comparing the level of said protein or proteins with the level of said protein or proteins in a reference sample,
wherein if the subject has dry eye or blepharitis, the level of at least one of the S100A4, S100A6, PLAA, GSTP1, JNK, Annexin 11 and Annexin 1 proteins is elevated with respect to its level in the reference sample and/or the level of at least one of the Cystatin S, Galectin 7, Lipophilin A and Peroxiredoxin 5 proteins is reduced with respect to its level in the reference sample.

In another aspect, the invention relates to an *in vitro* method for differentiating between dry eye and blepharitis in a subject which comprises:
(i) determining the level of one or more proteins selected from Galectin 7, Cystatin S, Cystatin N and S100A6 in a sample from a subject; and
(ii) comparing the level of said protein or proteins with the level of said protein/s in a reference sample,
wherein if the subject suffers from blepharitis, the Galectin 7 concentration is lower than 190 pg/µg, the S100A6 concentration is higher than 115 pg/µg and lower than 182 pg/µg, the Cystatin S concentration is higher than 98.5 pg/µg but lower than 118 pg/µg and/or the Cystatin N protein level is increased with respect to the concentration in a reference sample.

In another aspect, the invention relates to an *in vitro* method for diagnosing blepharitis and/or dry eye in a tear sample from a subject which comprises:
(i) determining the level of Galectin 7 in said sample; and
(ii) comparing the level of said protein with its level in a reference sample,
wherein the subject suffers from blepharitis if the Galectin 7 concentration is lower than 190 pg/µg and/or wherein the subject suffers from dry eye if the Galectin 7 concentration is lower than 257 pg/µg, but higher than 190 pg/µg.

In a last aspect, the invention relates to a kit comprising at least one reagent for the detection of at least one protein selected from the group of Lipophilin A, Peroxiredoxin 5, Cystatin S, Cystatin N, S100A4, S100A6, GSTP1, Annexin 1, JNK, Annexin 11, PLAA and Galectin 7.

### Brief Description of the Drawings

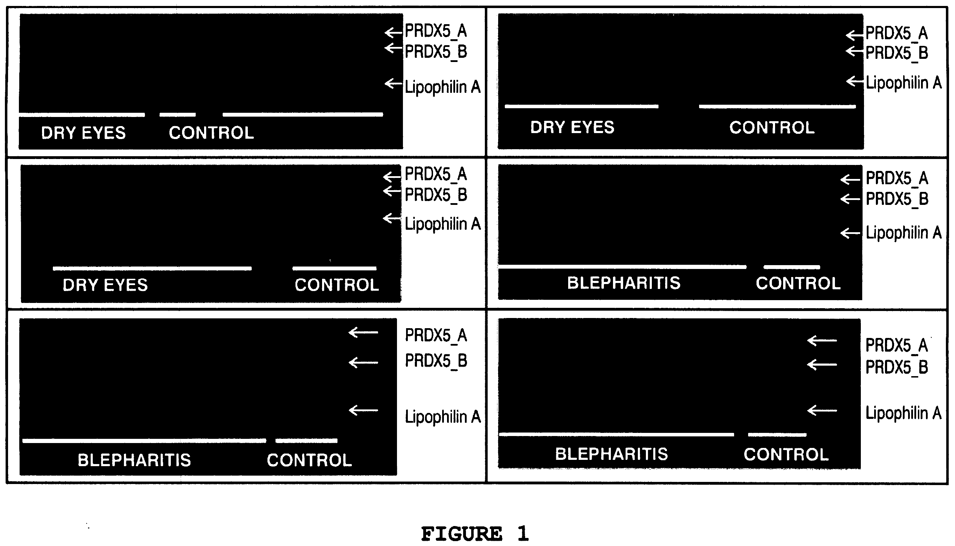

Figure 1 shows the validation by means of Immunofluorescent Multiplex Western Blot of Lipophilin A (lower band) and Peroxiredoxin 5 (upper bands, PRDX5 A and B), two of the candidate markers obtained by means of a literature search. In each case, the group of patients to which it corresponds is indicated.

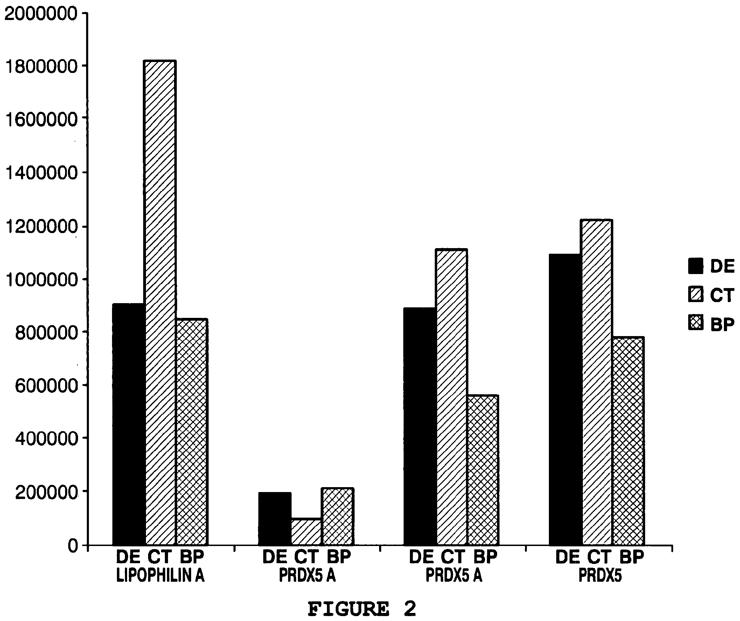

Figure 2 depicts a bar graph representing the normalized volume values for each group and for each marker. A reduction in the Lipophilin A concentration, both in dry eyes (DE) and in blepharitis (BP) with respect to the control (CT), can be observed. In the case of Peroxiredoxin 5, both in dry eyes and in blepharitis, the presence of an isoform (PRDX5A) which was virtually not present in the control group is observed.

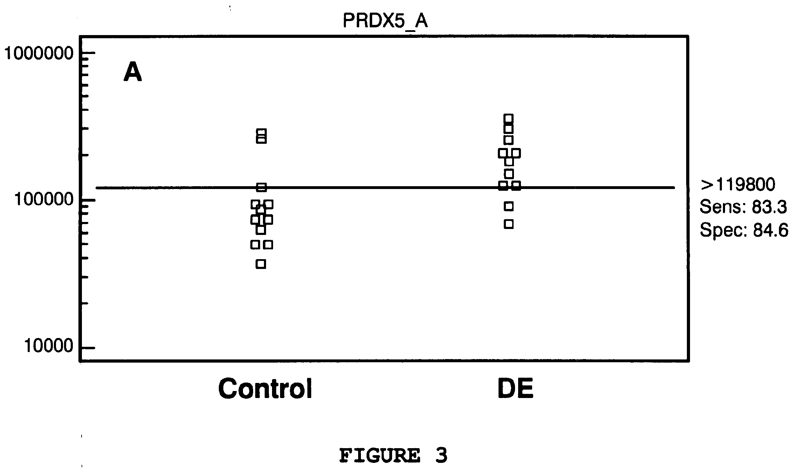

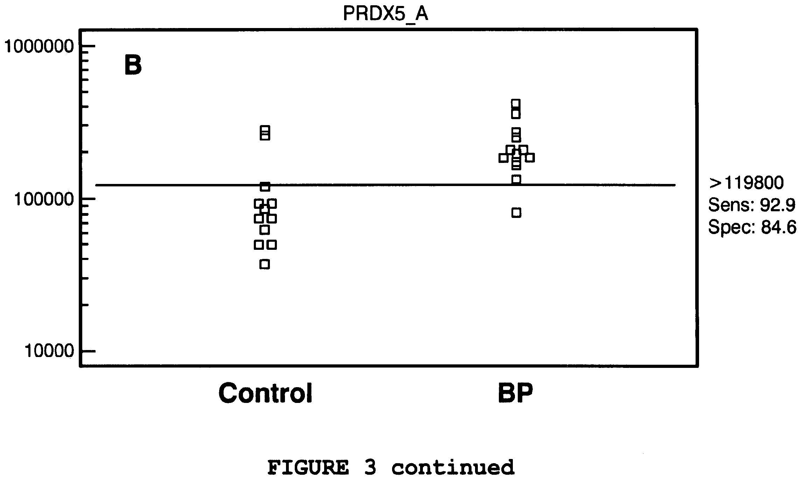

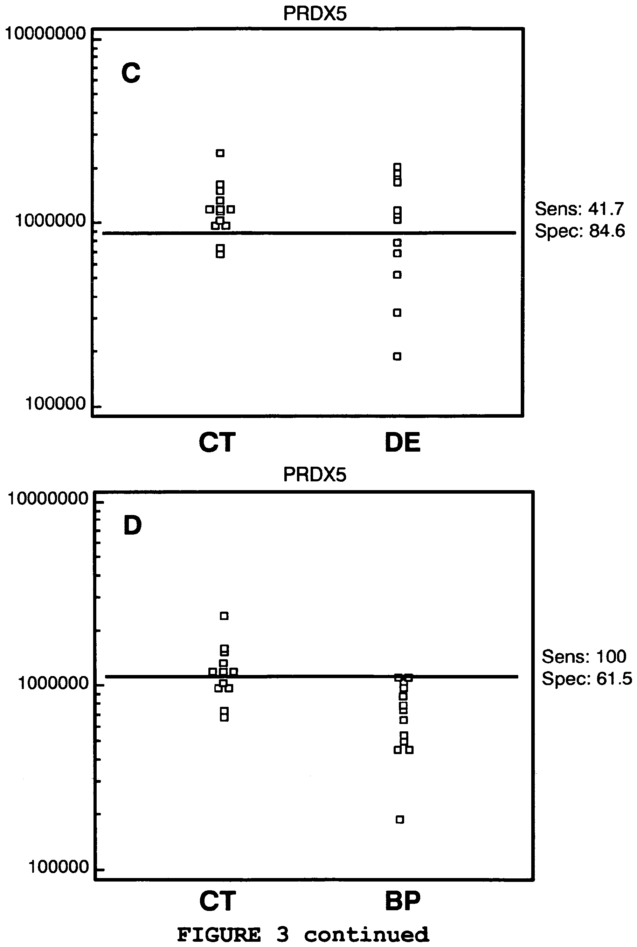

Figure 3 corresponds to a dot plot at a logarithmic scale which depicts the normalized volume values obtained for Peroxiredoxin 5A and Peroxiredoxin 5 (isoforms A+B) by means of Western blot. These graphs comparatively show dry eyes (DE) compared to a control (A) and blepharitis (BP) compared to a control (B) for the Peroxiredoxin 5A isoform. They also comparatively show dry eyes (DE) compared to a control (C) and blepharitis (BP) compared to a control (D) for Peroxiredoxin 5 (both isoforms). The x-axis shows, in the form of logical values, the pathological condition (independent variable) of the samples, whereas the y-axis shows the normalized volume values of said samples. The sensitivity and specificity values are indicated at the right of the plots (Sens and Spec, respectively).

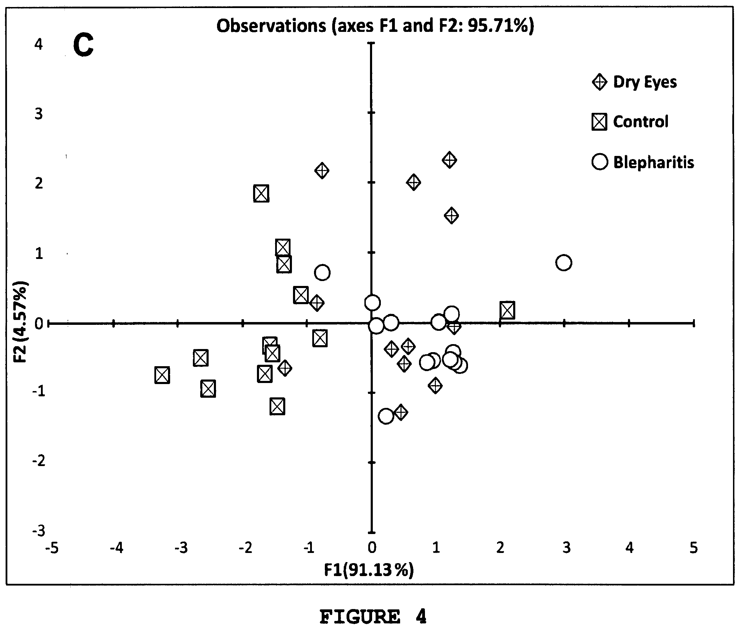

Figure 4 shows a graphical depiction of the discriminant analysis using Lipophilin A and Peroxiredoxin 5 (both isoforms). The samples from the control group are shown with a square (■), those of dry eyes with a rhombus (◆) and the samples from the blepharitis group with a circle (●).

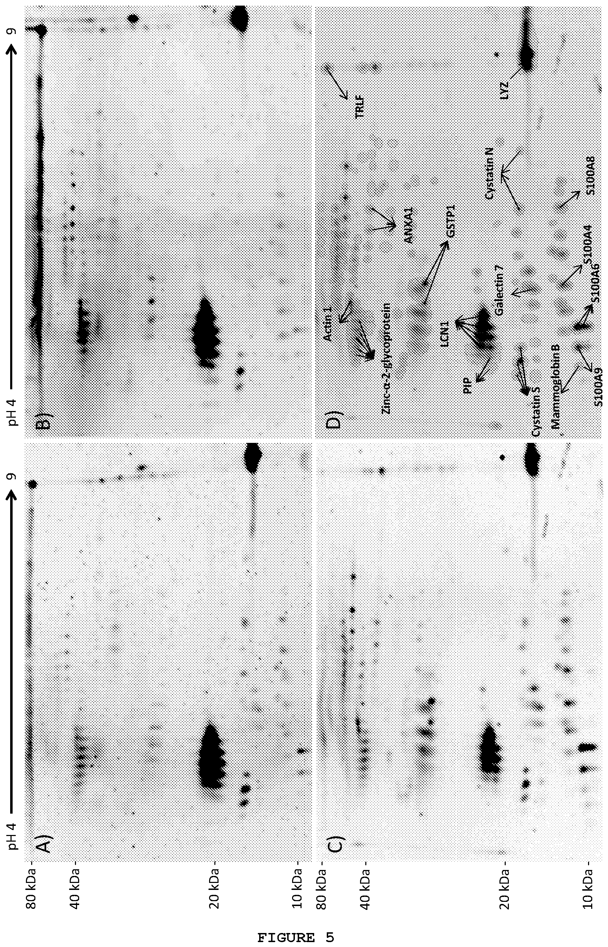

Figure 5 shows the tear proteome of a dry eyes (A), blepharitis (B) and control (C) patient. The tear partial proteome is also shown, indicating the spots identified by MALDI-TOF/TOF (D). A group of spots is detected corresponding to multiple isoforms of the same proteins. The 2-D electrophoresis has been performed in 17 cm strips with a pH range of 4-9 loading 40 µg of total tear protein of each patient, after its treatment with a Sigma Seppro column and an isoelectric focusing of 60623 Vxh. The second dimension has been performed in 15% polyacrylamide gels, which allows a separation between 10 and 250 kDa. The fluorescent reagent Sypro Ruby was used for the viewing and the VersaDoc system was used as the fluorescence detection system.

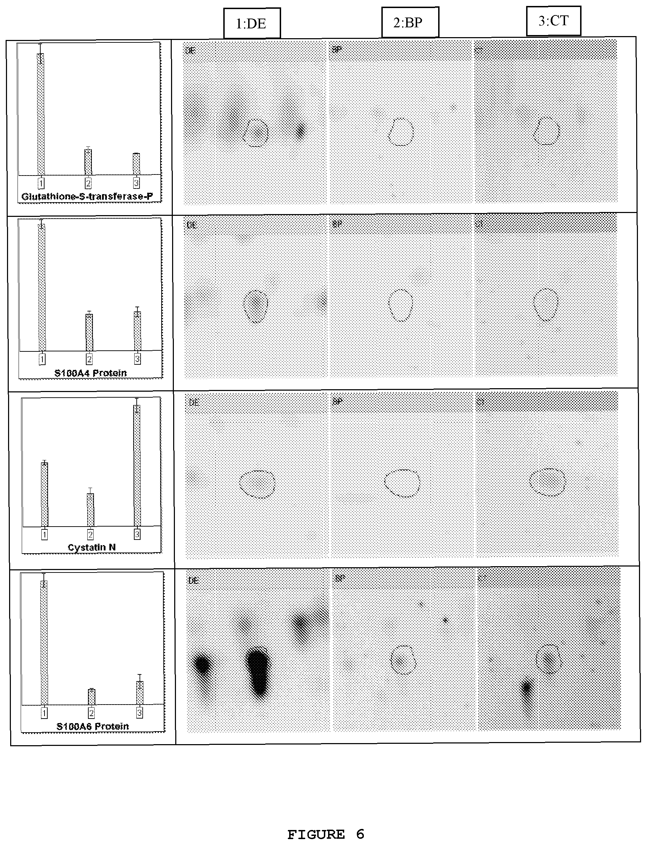

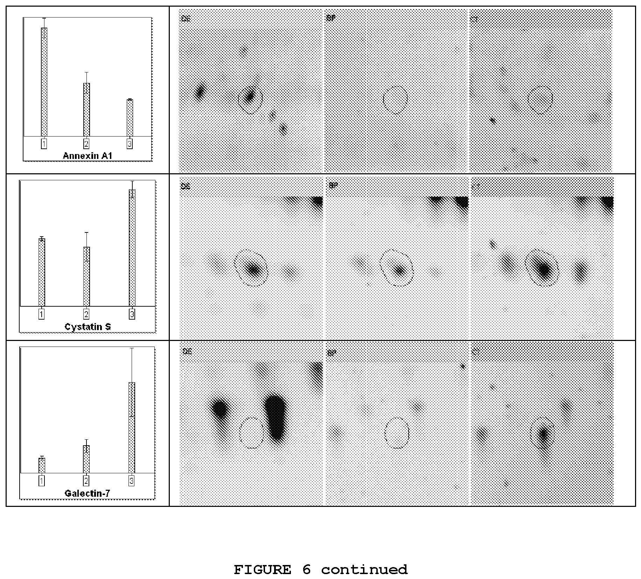

Figure 6 shows a proteome with the differential expression of the spots with the highest statistically significant variance of the study. At the left of each of the spots, the mean expression levels for each of the groups are depicted in the form of a bar graph, 1 being the dry eye group, 2 the blepharitis group and 3 the control group, together with the name of the protein identified by means of MALDI-TOF/TOF mass spectrometry.

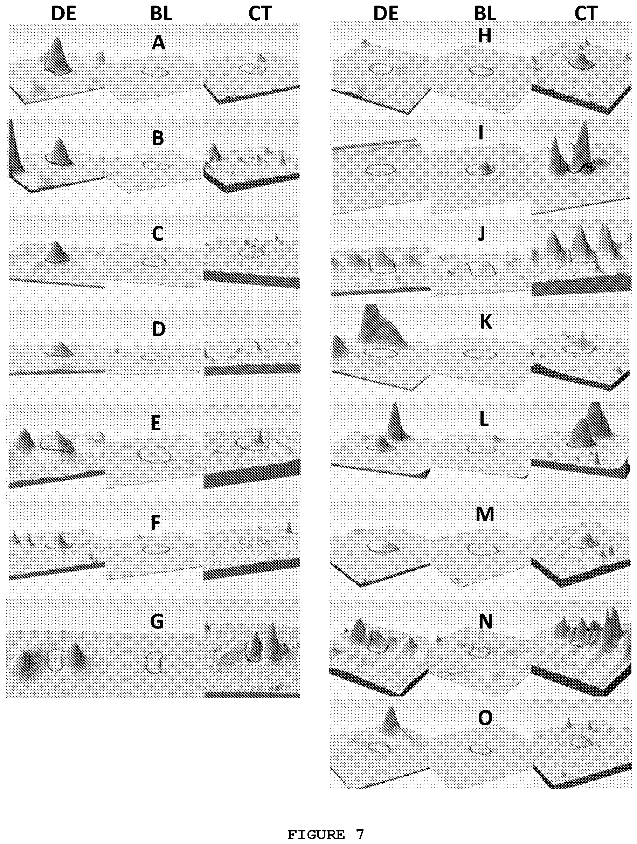

Figure 7 shows a tridimensional representation of the more representative spots corresponding to the proteins S100A6 (A), S100A9 (B) S100A8 (C), S100A4 (D), Glutathion S-transferase-P1 (GSTP1) (E), Annexin A1 (ANXA1) (F), Lipocalin-1 (LCN1) (G). Prolactin inducible protein (PIP) (H), Lactotransferrin (LTF) (I), Zinc-alpha-2-glycoprotein (AZGP1) (J), Galectin-7 (LEG7) (K), Cystatin-S (CST4) (L), Cystatin-SN (CST1) (M), Actin B (ACTB) (N) and Mammaglobin B (SG2A1) (O). The volume of each spot is determined by the intensity of the signal obtained when staining the bidimensional gels with SyproRuby by its area.

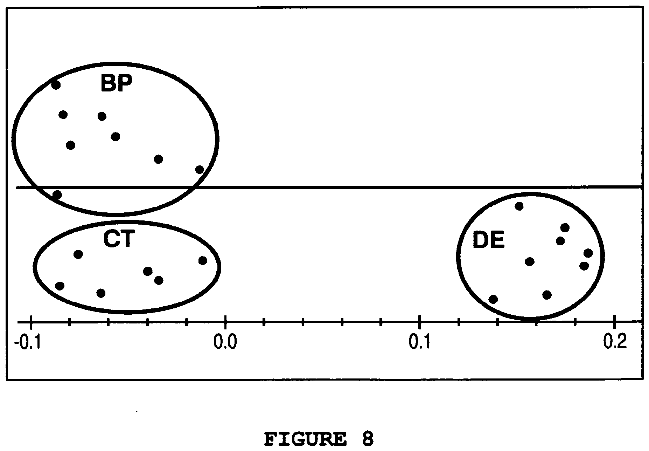

Figure 8 shows a depiction of the analysis of principal components using only those spots which are statistically significant in the comparison between the groups, i.e., 54 spots within the proteome with a statistical power greater than 0.9. The proteins identified as Group 1 in Table 5 are included within these spots. Each spot in the graph represents a gel within the study included in one of the following groups: DE: dry eyes; BP: blepharitis; CT: control. The x-axis shows the principal component 1 (PC1), which represents 73% of the variance, whereas the y-axis shows the principal component 2 (PC2) with 17.5% of the variance. Therefore, 90.5% of the variance between the groups of the study is explained between the first two principal components (PC1 and PC2). A perfect separation between the different groups can be observed, which indicates that the significantly altered proteins can be good diagnostic candidates to be able to perform a differentiation between the groups.

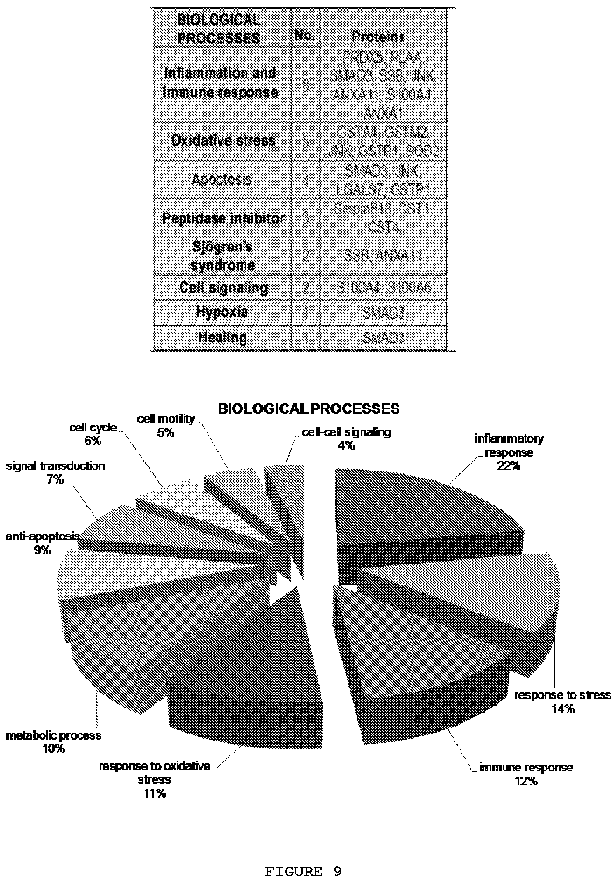

Figure 9 shows a diagram of the markers of the invention and their involvement in different biological processes. The table shows the main biological processes in which each of the biomarker candidates obtained in this study are involved, together with their abundance. The percentages of frequency of the most abundant biological processes are schematized below by means of a circular depiction of proportions.

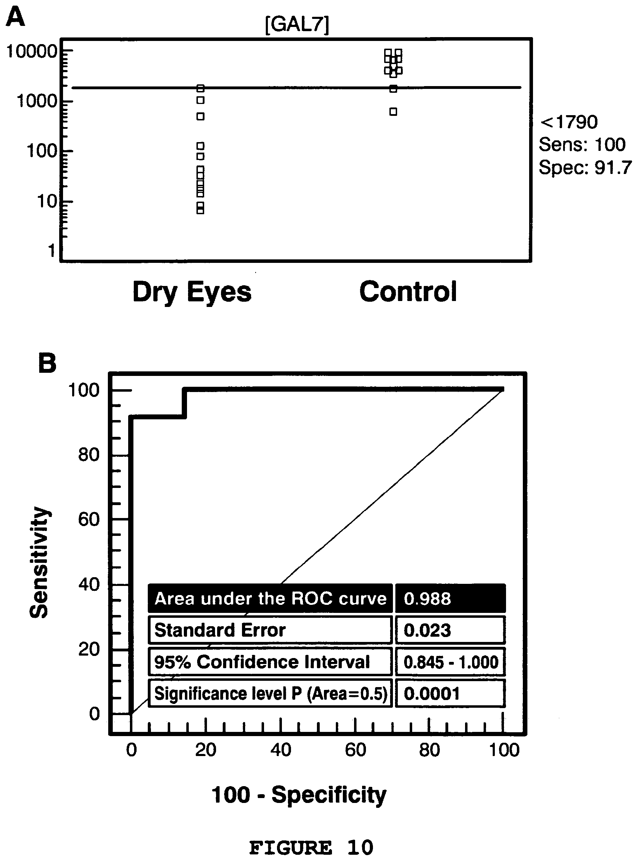

Figure 10 shows a dot plot at a logarithmic scale (A) in which the Galectin 7 concentration values obtained for the control group and for the pathological dry eye group are compared. The critical point of Galectin 7 concentration is 1.790 pg/ml, which indicates that below said value it has 91.7% specificity and 100% sensitivity. (B) ROC curve for the Galectin 7 concentration values obtained from the ELISA assays. An area under the curve of 0.988 is verified, indicating that said marker could behave like an excellent diagnostic tool for the separation between the control and dry eyes patient groups.

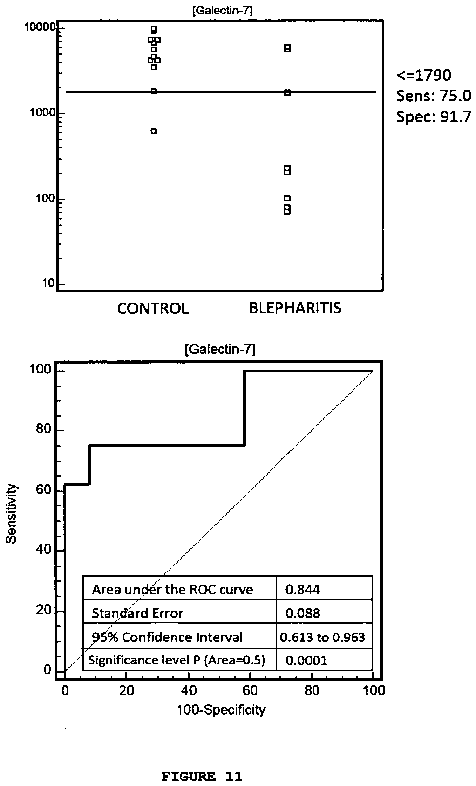

Figure 11 shows a dot plot at a logarithmic scale (A) in which the Galectin 7 concentration values obtained for the control group and for the pathological blepharitis group are compared. The critical point of Galectin 7 concentration is 1.790 pg/ml, which indicates that below said value it has 75% sensitivity and 91.7% specificity. (B) ROC curve for the Galectin 7 concentration values obtained from the ELISA assays. An area under the curve of 0.844 is verified, indicating that said marker could behave like an excellent diagnostic tool for the separation between the control and blepharitis patient groups.

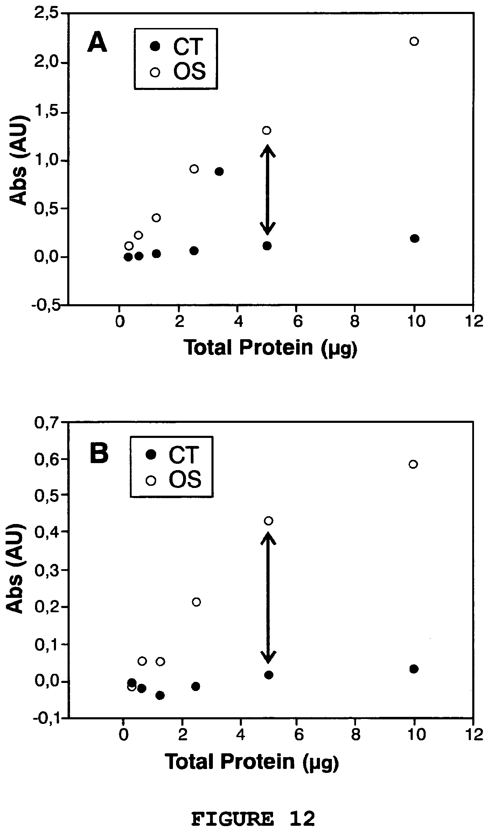

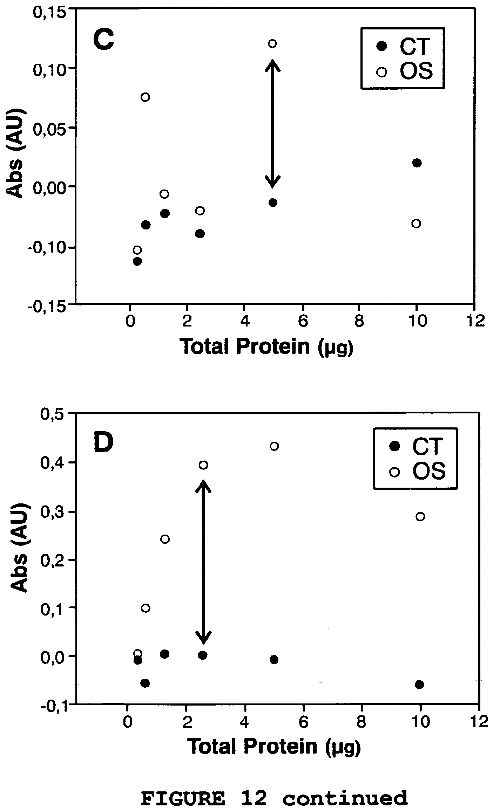

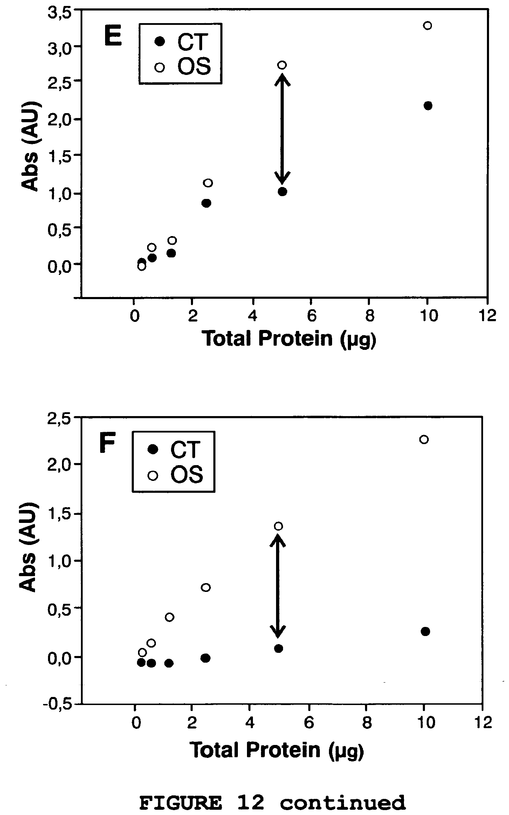

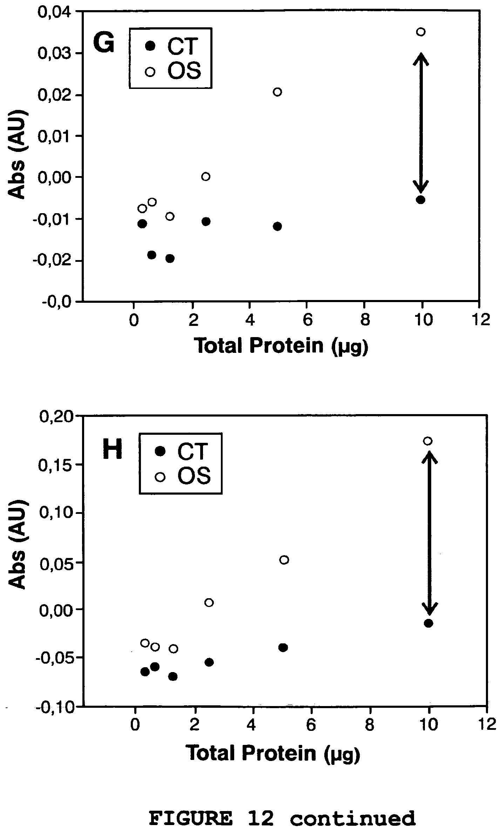

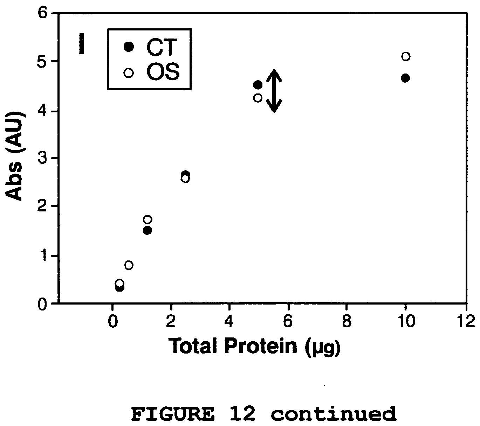

Figure 12 shows a validation screening using sandwich ELISA between the dry eye group and the control group for nine of the proteins which showed greater expression changes, or which were established as putative markers according with the expression network analysis. (A) ANXA1, (B) PLAA, (C) SOD2, (D) ANXA11, (E) PRDX5, (F) S100A6, (G) GSTP1, (H) GSTM2, (I) CST4. The images represent the absorbance value (y-axis) against the total protein amount studied (x-axis) for each marker and each group. The total protein amount needed to distinguish between groups and the maximum signal to noise relation for each marker is indicated with an arrow. Black dots represent the control group while white dots represent the dry eye group.

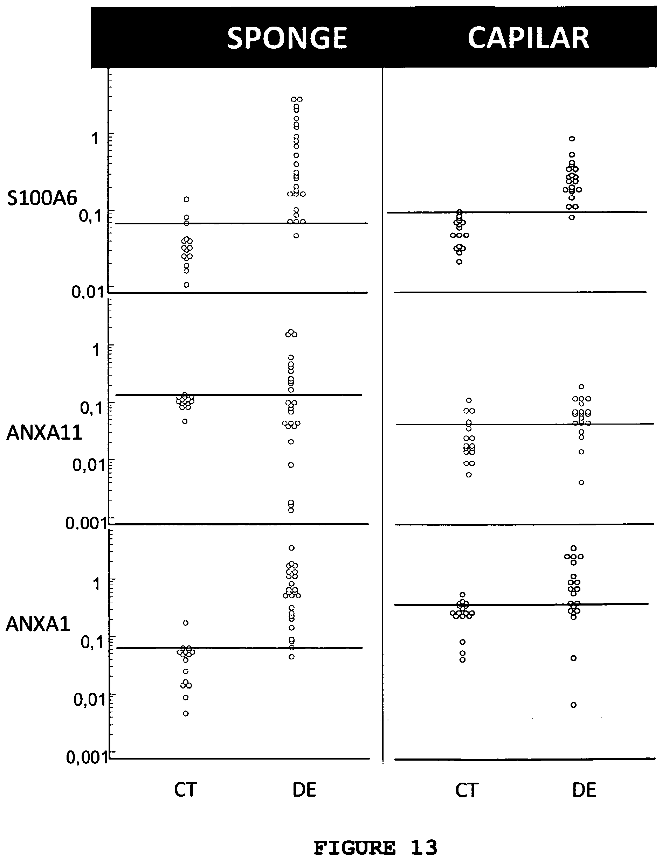

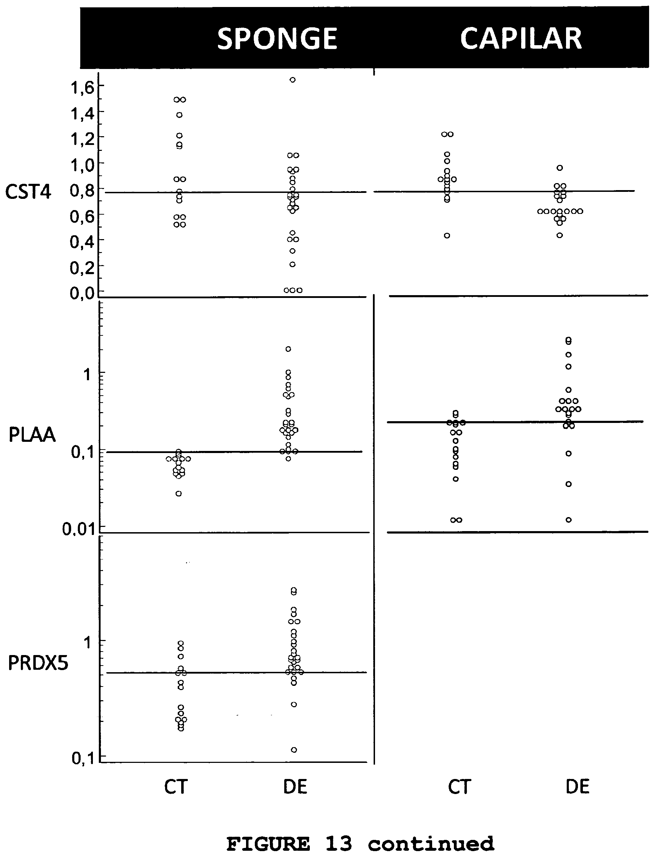

Figure 13 corresponds to a dot plot at a logarithmic scale which depicts the S100A6, ANXA11, ANXA1, CST4, PLAA and PRDX5 proteins concentration values with respect to the total protein concentration (pg/µg), obtained for the control group and for the pathological dry eye group. The individual results for the two validation steps are shown for each protein.

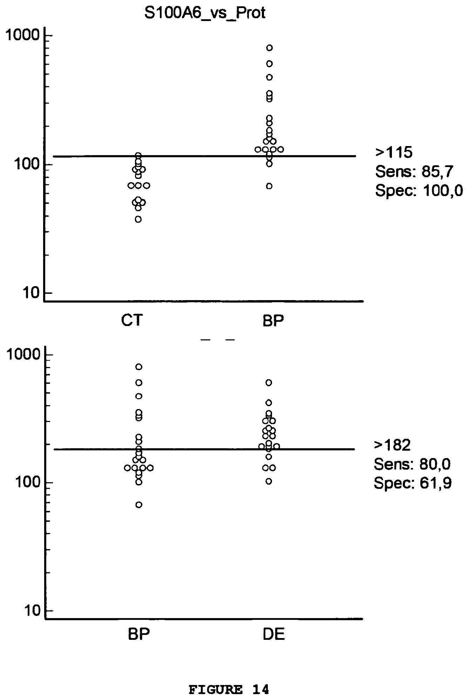

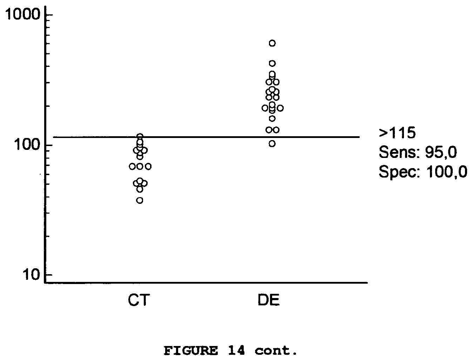

Figure 14 shows a dot plot at a logarithmic scale which depicts the S100A6 protein concentration value with respect to the total protein concentration (pg/µg), obtained for the control group vs blepharitis group, blepharitis group vs dry eye group and control group vs dry eye group, for validation 2.

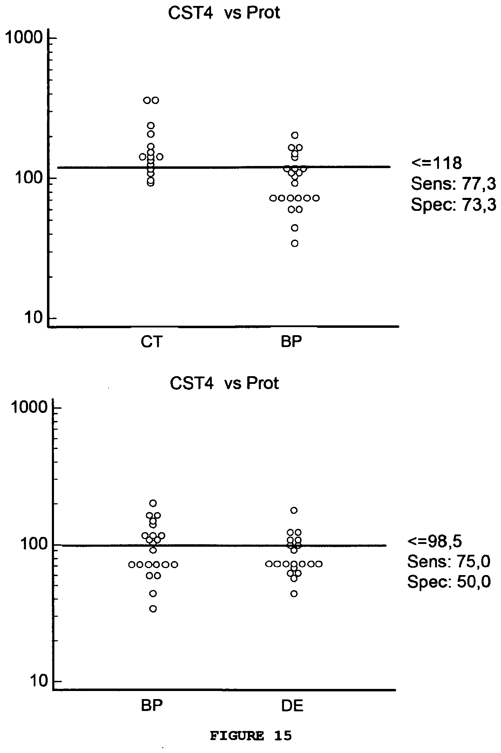

Figure 15 shows a dot plot at a logarithmic scale which depicts the CST4 protein concentration value with respect to the total protein concentration (pg/µg), obtained for the control group vs blepharitis group and for blepharitis group vs dry eye group for validation 2.

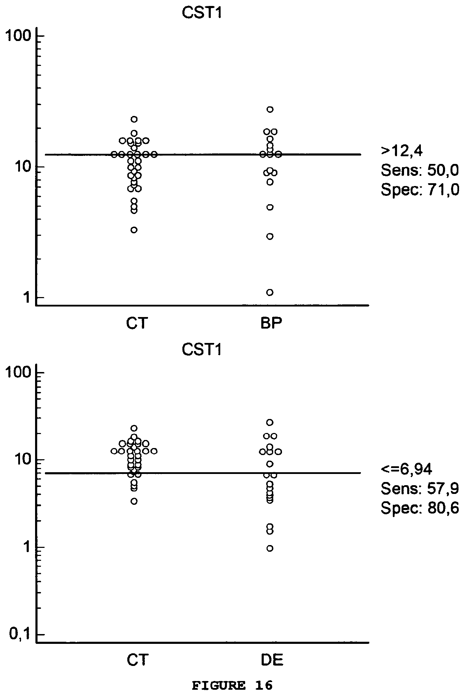

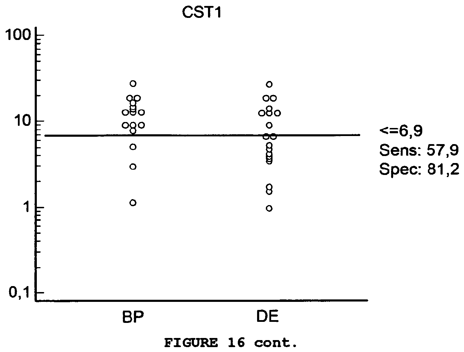

Figure 16 shows a dot plot at a logarithmic scale which depicts the CST1 protein concentration value with respect to the total protein concentration (pg/µg), obtained for the control group vs blepharitis group, the control group vs dry eye group and for blepharitis group vs dry eye group, for validation 2.

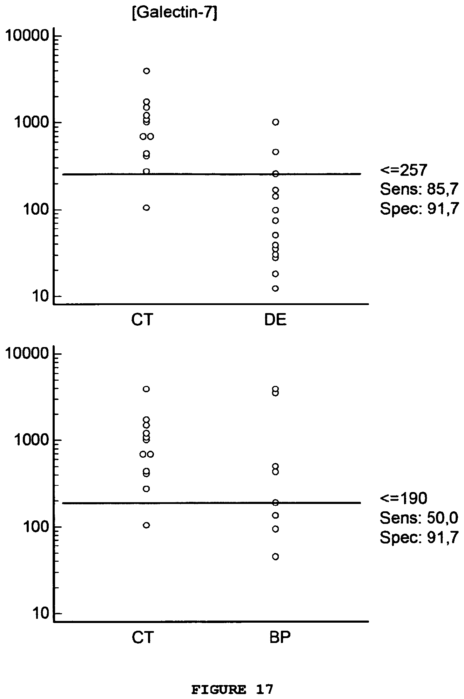

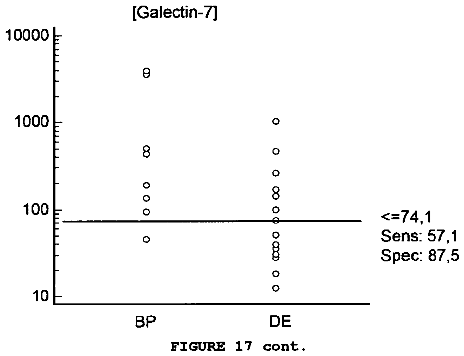

Figure 17 shows a dot plot at a logarithmic scale which depicts the Galectin-7 protein concentration value with respect to the total protein concentration (pg/µg), obtained for the control group vs dry eye group, the control group vs blepharitis group and for blepharitis group vs dry eye group, for validation 2.

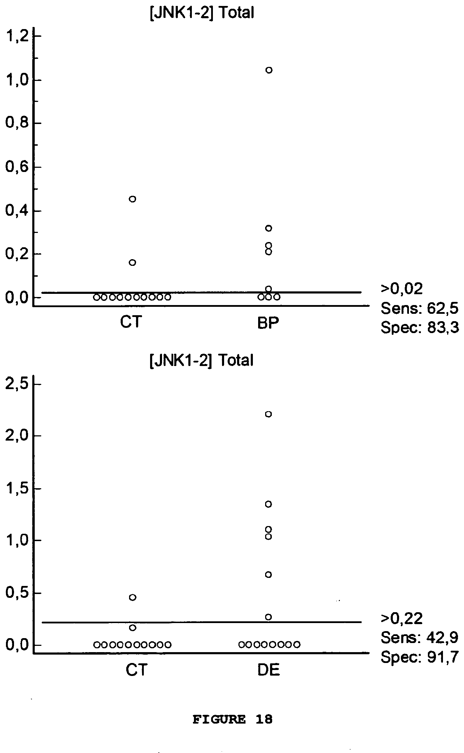

Figure 18 shows a dot plot at a logarithmic scale which depicts the JNK protein concentration value with respect to the total protein concentration (pg/µg), obtained for the control group vs blepharitis group and the control group vs dry eye group, for validation 2.

### Detailed Description of the Invention

The inventors of the present invention have discovered a new group of molecular markers which allow the detection and/or the diagnosis of dry eye and blepharitis and, furthermore, allow discriminating between patients suffering from dry eye and blepharitis.

In a first aspect, the invention relates to an *in vitro* method for diagnosing dry eye in a subject which comprises:
(i) determining the level of S100A6 protein in a sample from a subject; and
(ii) comparing the level of said protein with
   the level of said protein in a reference sample,
wherein if the subject has dry eye, the level of the S100A6 protein is elevated with respect to its level in the reference sample.
In a particular embodiment, the first method of the invention additionally comprises determining the level of one or more proteins selected from the group of PLAA, Serpin B13, Reticulocalbin 1, Superoxide dismutase, SMAD3, GSTM2, GSTA4, Cystatin S and SSB.

It is also disclosed that the S100A6 protein can individually serve as a marker for the diagnosis of dry eye in a tear sample from a subject. In subjects with dry eyes, it is observed that the level of the S100A6 protein is elevated with respect to the level of said protein in a reference sample. The concentration level of this protein standardized with respect to the amount of total protein shows a high efficiency in the diagnosis of dry eye pathology, with correct assignment percentages of 91.45%, as shown in Table 8. It is disclosed that if the protein concentration level is higher than 182 pg/µg, the subject suffers from dry eye.

It is also disclosed that Annexin 1 protein can individually serve as a marker for the diagnosis of dry eye in a tear sample from a subject. In subjects with dry eyes, it is observed that the level of the Annexin 1 protein is elevated with respect to the level of said protein in a reference sample. The concentration level of this protein standardized with respect to the amount of total protein shows a high efficiency in the diagnosis of dry eye pathology, with correct assignment percentages of 92.80%, as shown in Table 8.

It is also disclosed that the PLAA protein can individually serve as a marker for the diagnosis of dry eye in a tear sample from a subject. In subjects with dry eyes, it is observed that the level of the PLAA protein is elevated with respect to the level of said protein in a reference sample. The concentration level of this protein standardized with respect to the amount of total protein shows a high efficiency in the diagnosis of dry eye pathology, with correct assignment percentages of 98.10%, as shown in Table 8.

It is also disclosed that the Galectin 7 can individually serve as a marker for the diagnosis of dry eye in a tear sample from a subject. The subject suffers from dry eye if the Galectin 7 protein concentration is reduced with respect to the said concentration in the reference sample. Preferably, the subject suffers from dry eye if the Galectin 7 concentration is lower than 257 pg/µg, but higher than 190 pg/µg, with respect to the reference sample.

It is also disclosed that the CST4 (Cystatin 4 or Cystatin S) can individually serve as a marker for the diagnosis of dry eye in a tear sample from a subject. The subject suffers from dry eye if the CST4 protein concentration is reduced with respect to the said concentration in the reference sample. Preferably, the subject suffers from dry eye if the concentration of CST4 protein is lower than 98.5 pg/µg, with respect to a reference sample, the subject suffers from dry eye.

It is also disclosed that the CST1 (Cystatin 1 or Cystatin N) can individually serve as a marker for the diagnosis of dry eye in a tear sample from a subject. The subject suffers from dry eye if the CST1 protein concentration is reduced with respect to the said concentration in the reference sample. Preferably, the subject suffers from dry eye if the concentration of CST4 protein is lower than 7 pg/µg, with respect to a reference sample, the subject suffers from dry eye.

It is also disclosed that markers useful for diagnosing dry eye are: ANXA1, GSTP1, S100A8, S100A9, S100A4 and S100A6. Thus, when the expression level of one or more of these markers is elevated with respect to its level in the reference sample, it is indicative of the subject having dry eye.

It is also disclosed that markers useful for diagnosing dry eye are: S100A6, CST4, ANXA1, ANXA11 and PLAA. Thus, when the expression level of one or more of the following markers S100A6, ANXA1, ANXA11 and PLAA is elevated and a reduction in CST4, with respect to its level in the reference sample, it is indicative of the subject having dry eye.

It is also disclosed that the combination of S100A6, ANXA1, GSTP1, AZGP1, ACTB and LEG7 (Galectin 7) proteins or each of the proteins individually are useful for the diagnosis of dry eye.

Each of these markers described above can individually serve as a marker for diagnosis of dry eye. However, combinations of these markers are also possible. Although not forming part of the present invention, useful combinations of these markers include the following combinations of 2, 3, 4, 5, 6 and 7 markers:

| | | | | |
|---|---|---|---|---|
| S100A6, CST1 | S100A6, CST1, ANXA1 | CST1, ANXA11, PRDX5 | S100A6, CST1, ANXA1, ANXA11 | S100A6, ANXA11, GSTP1, PRDX5 |
| S100A6, ANXA1 | S100A6, CST1, ANXA11 | CST1, ANXA11, GAL7 | S100A6, CST1, ANXA1, PLAA | S100A6, ANXA11, GSTP1, GAL7 |
| S100A6, ANXA11 | S100A6, CST1, PLAA | CST1, PLAA, GSTP1 | S100A6, CST1, ANXA1, GSTP1 | S100A6, ANXA11, PRDX5, GAL7 |
| S100A6, PLAA | S100A6, CST1, GSTP1 | CST1, PLAA, PRDX5 | S100A6, CST1, ANXA1, PRDX5 | S100A6, PLAA, GSTP1, PRDX5 |
| S100A6, GSTP1 | S100A6, CST1, PRDX5 | CST1, PLAA, GAL7 | S100A6, CST1, ANXA1, GAL7 | S100A6, PLAA, GSTP1, GAL7 |
| S100A6, PRDX5 | S100A6, CST1, GAL7 | CST1, GSTP1, PRDX5 | S100A6, CST1, ANXA11, PLAA | S100A6, PLAA, PRDX5, GAL7 |
| S100A6, GAL7 | S100A6, ANXA1, ANXA11 | CST1, GSTP1, GAL7 | S100A6, CST1, ANXA11, GSTP1 | S100A6, GSTP1, PRDX5, GAL7 |
| CST1, ANXA1 | S100A6, ANXA1, PLAA | CST1, PRDX5, GAL7 | S100A6, CST1, ANXA11, PRDX5 | CST1, ANXA1, ANXA11, PLAA |
| CST1, ANXA11 | S100A6, ANXA1, GSTP1 | ANXA1, ANXA11, PLAA | S100A6, CST1, ANXA11, GAL7 | CST1, ANXA1, ANXA11, GSTP1 |
| CST1, PLAA | S100A6, ANXA1, PRDX5 | ANXA1, ANXA11, GSTP1 | S100A6, CST1, PLAA, GSTP1 | CST1, ANXA1, ANXA11, PRDX5 |
| CST1, GSTP1 | S100A6, ANXA1, GAL7 | ANXA1, ANXA11, PRDX5 | S100A6, CST1, PLAA, PRDX5 | CST1, ANXA1, ANXA11, GAL7 |
| CST1, PRDX5 | S100A6, ANXA11, PLAA | ANXA1, ANXA11, GAL7 | S100A6, CST1, PLAA, GAL7 | CST1, ANXA1, PLAA, GSTP1 |
| CST1, GAL7 | S100A6, ANXA11, GSTP1 | ANXA1, PLAA, GSTP1 | S100A6, CST1, GSTP1, PRDX5 | CST1, ANXA1, PLAA, PRDX5 |
| ANXA1, ANXA11 | S100A6, ANXA11, PRDX5 | ANXA1, PLAA, PRDX5 | S100A6, CST1, GSTP1, GAL7 | CST1, ANXA1, PLAA, GAL7 |
| ANXA1, PLAA | S100A6, ANXA11, GAL7 | ANXA1, PLAA, GAL7 | S100A6, CST1, PRDX5, GAL7 | CST1, ANXA1, GSTP1, PRDX5 |
| ANXA1, GSTP1 | S100A6, PLAA, GSTP1 | ANXA1, GSTP1, PRDX5 | S100A6, ANXA1, ANXA11, PLAA | CST1, ANXA1, GSTP1, GAL7 |
| ANXA1, PRDX5 | S100A6, PLAA, PRDX5 | ANXA1, GSTP1, GAL7 | S100A6, ANXA1, ANXA11, GSTP1 | CST1, ANXA1, PRDX5, GAL7 |
| ANXA1, GAL7 | S100A6, PLAA, GAL7 | ANXA1, PRDX5, GAL7 | S100A6, ANXA1, ANXA11, PRDX5 | CST1, ANXA11, PLAA, GSTP1 |
| ANXA11, PLAA | S100A6, GSTP1, PRDX5 | ANXA11, PLAA, GSTP1 | S100A6, ANXA1, ANXA11, GAL7 | CST1, ANXA11, PLAA, PRDX5 |
| ANXA11, GSTP1 | S100A6, GSTP1, GAL7 | ANXA11, PLAA, PRDX5 | S100A6, ANXA1, PLAA, GSTP1 | CST1, ANXA11, PLAA, GAL7 |
| ANXA11, PRDX5 | S100A6, PRDX5, GAL7 | ANXA11, PLAA, GAL7 | S100A6, ANXA1, PLAA, PRDX5 | CST1, ANXA11, GSTP1, PRDX5 |
| ANXA11, GAL7 | CST1, ANXA1, ANXA11 | ANXA11, GSTP1, PRDX5 | S100A6, ANXA1, PLAA, GAL7 | CST1, ANXA11, GSTP1, GAL7 |
| PLAA, GSTP1 | CST1, ANXA1, PLAA | ANXA11, GSTP1, GAL7 | S100A6, ANXA1, GSTP1, PRDX5 | CST1, ANXA11, PRDX5, GAL7 |
| PLAA, PRDX5 | CST1, ANXA1, GSTP1 | ANXA11, PRDX5, GAL7 | S100A6, ANXA1, GSTP1, GAL7 | CST1, PLAA, GSTP1, PRDX5 |
| PLAA, GAL7 | CST1, ANXA1, PRDX5 | PLAA, GSTP1, PRDX5 | S100A6, ANXA1, PRDX5, GAL7 | CST1, PLAA, GSTP1, GAL7 |
| GSTP1, PRDX5 | CST1, ANXA1, GAL7 | PLAA, GSTP1, GAL7 | S100A6, ANXA11, PLAA, GSTP1 | CST1, PLAA, PRDX5, GAL7 |
| GSTP1, GAL7 | CST1, ANXA11, PLAA | PLAA, PRDX5, GAL7 | S100A6, ANXA11, PLAA, PRDX5 | CST1, GSTP1, PRDX5, GAL7 |
| PRDX5, GAL7 | CST1, ANXA11, GSTP1 | GSTP1, PRDX5, GAL7 | S100A6, ANXA11, PLAA, GAL7 | ANXA1, ANXA11, PLAA, GSTP1 |

| | | | |
|---|---|---|---|
| ANXA1, ANXA11, PLAA, PRDX5 | S100A6, CST1, PLAA, PRDX5, GAL7 | ANXA1, ANXA11, PLAA, GSTP1, PRDX5 | CST1, ANXA11, PLAA, GSTP1, PRDX5, GAL7 |
| ANXA1, ANXA11, PLAA, GAL7 | S100A6, CST1, GSTP1, PRDX5, GAL7 | ANXA1, ANXA11, PLAA, GSTP1, GAL7 | ANXA1, ANXA11, PLAA, GSTP1, PRDX5, GAL7 |
| ANXA1, ANXA11, GSTP1, PRDX5 | S100A6, ANXA1, ANXA11, PLAA, GSTP1 | ANXA1, ANXA11, PLAA, PRDX5, GAL7 | S100A6, CST1, ANXA1, ANXA11, PLAA, GSTP1, PRDX5 |
| ANXA1, ANXA11, GSTP1, GAL7 | S100A6, ANXA1, ANXA11, PLAA, PRDX5 | ANXA1, ANXA11, GSTP1, PRDX5, GAL7 | S100A6, CST1, ANXA1, ANXA11, PLAA, GSTP1, GAL7 |
| ANXA1, ANXA11, PRDX5, GAL7 | S100A6, ANXA1, ANXA11, PLAA, GAL7 | ANXA1, PLAA, GSTP1, PRDX5, GAL7 | S100A6, CST1, ANXA1, ANXA11, PLAA, PRDX5, GAL7 |
| ANXA1, PLAA, GSTP1, PRDX5 | S100A6, ANXA1, ANXA11, GSTP1, PRDX5 | ANXA11, PLAA, GSTP1, PRDX5, GAL7 | S100A6, CST1, ANXA1, ANXA11, GSTP1, PRDX5, GAL7 |
| ANXA1, PLAA, GSTP1, GAL7 | S100A6, ANXA1, ANXA11, GSTP1, GAL7 | S100A6, CST1, ANXA1, ANXA11, PLAA, GSTP1 | S100A6, CST1, ANXA1, PLAA, GSTP1, PRDX5, GAL7 |
| ANXA1, PLAA, PRDX5, GAL7 | S100A6, ANXA1, ANXA11, PRDX5, GAL7 | S100A6, CST1, ANXA1, ANXA11, PLAA, PRDX5 | S100A6, CST1, ANXA11, PLAA, GSTP1, PRDX5, GAL7 |
| ANXA1, GSTP1, PRDX5, GAL7 | S100A6, ANXA1, PLAA, GSTP1, PRDX5 | S100A6, CST1, ANXA1, ANXA11, PLAA, GAL7 | S100A6, ANXA1, ANXA11, PLAA, GSTP1, PRDX5, GAL7 |
| ANXA11, PLAA, GSTP1, PRDX5 | S100A6, ANXA1, PLAA, GSTP1, GAL7 | S100A6, CST1, ANXA1, ANXA11, GSTP1, PRDX5 | CST1, ANXA1, ANXA11, PLAA, GSTP1, PRDX5, GAL7 |
| ANXA11, PLAA, GSTP1, GAL7 | S100A6, ANXA1, PLAA, PRDX5, GAL7 | S100A6, CST1, ANXA1, ANXA11, GSTP1, GAL7 | CST1, ANXA1, ANXA11, GSTP1, PRDX5, GAL7 |
| ANXA11, PLAA, PRDX5, GAL7 | S100A6, ANXA1, GSTP1, PRDX5, GAL7 | S100A6, CST1, ANXA1, ANXA11, PRDX5, GAL7 | CST1, ANXA1, PLAA, GSTP1, PRDX5, GAL7 |
| ANXA11, GSTP1, PRDX5, GAL7 | S100A6, ANXA11, PLAA, GSTP1, PRDX5 | S100A6, CST1, ANXA1, PLAA, GSTP1, PRDX5 | CST1, ANXA11, GSTP1, PRDX5, GAL7 |
| PLAA, GSTP1, PRDX5, GAL7 | S100A6, ANXA11, PLAA, GSTP1, GAL7 | S100A6, CST1, ANXA1, PLAA, GSTP1, GAL7 | CST1, PLAA, GSTP1, PRDX5, GAL7 |
| S100A6, CST1, ANXA1, ANXA11, PLAA | S100A6, ANXA11, PLAA, PRDX5, GAL7 | S100A6, CST1, ANXA1, PLAA, PRDX5, GAL7 | S100A6, CST1, PLAA, GSTP1, PRDX5 |
| S100A6, CST1, ANXA1, ANXA11, GSTP1 | S100A6, ANXA11, GSTP1, PRDX5, GAL7 | S100A6, CST1, ANXA1, GSTP1, PRDX5, GAL7 | S100A6, CST1, PLAA, GSTP1, GAL7 |
| S100A6, CST1, ANXA1, ANXA11, PRDX5 | S100A6, PLAA, GSTP1, PRDX5, GAL7 | S100A6, CST1, ANXA11, PLAA, GSTP1, PRDX5 | CST1, ANXA1, ANXA11, PLAA, GSTP1, GAL7 |
| S100A6, CST1, ANXA1, ANXA11, GAL7 | CST1, ANXA1, ANXA11, PLAA, GSTP1 | S100A6, CST1, ANXA11, PLAA, GSTP1, GAL7 | CST1, ANXA1, ANXA11, PLAA, PRDX5, GAL7 |
| S100A6, CST1, ANXA1, PLAA, GSTP1 | CST1, ANXA1, ANXA11, PLAA, PRDX5 | S100A6, CST1, ANXA11, PLAA, PRDX5, GAL7 | CST1, ANXA11, PLAA, GSTP1, GAL7 |
| S100A6, CST1, ANXA1, PLAA, PRDX5 | CST1, ANXA1, ANXA11, PLAA, GAL7 | S100A6, CST1, ANXA11, GSTP1, PRDX5, GAL7 | CST1, ANXA11, PLAA, PRDX5, GAL7 |
| S100A6, CST1, ANXA1, PLAA, GAL7 | CST1, ANXA1, ANXA11, GSTP1, PRDX5 | S100A6, CST1, PLAA, GSTP1, PRDX5, GAL7 | S100A6, CST1, ANXA11, GSTP1, GAL7 |
| S100A6, CST1, | CST1, ANXA1, ANXA11, | S100A6, ANXA1, | S100A6, CST1, |
| ANXA1, GSTP1, PRDX5 | GSTP1, GAL7 | ANXA11, PLAA, GSTP1, PRDX5 | ANXA11, PRDX5, GAL7 |
| S100A6, CST1, ANXA1, GSTP1, GAL7 | CST1, ANXA1, ANXA11, PRDX5, GAL7 | S100A6, ANXA1, ANXA11, PLAA, GSTP1, GAL7 | S100A6, ANXA11, PLAA, GSTP1, PRDX5, GAL7 |
| S100A6, CST1, ANXA1, PRDX5, GAL7 | CST1, ANXA1, PLAA, GSTP1, PRDX5 | S100A6, ANXA1, ANXA11, PLAA, PRDX5, GAL7 | CST1, ANXA1, ANXA11, PLAA, GSTP1, PRDX5 |
| S100A6, CST1, ANXA11, PLAA, GSTP1 | CST1, ANXA1, PLAA, GSTP1, GAL7 | S100A6, ANXA1, ANXA11, GSTP1, PRDX5, GAL7 | CST1, ANXA1, GSTP1, PRDX5, GAL7 |
| S100A6, CST1, ANXA11, PLAA, PRDX5 | CST1, ANXA1, PLAA, PRDX5, GAL7 | S100A6, ANXA1, PLAA, GSTP1, PRDX5, GAL7 | CST1, ANXA11, PLAA, GSTP1, PRDX5 |
| S100A6, CST1, ANXA11, GSTP1, PRDX5 | S100A6, CST1, ANXA11, PLAA, GAL7 | | |

It is also disclosed an *in vitro* method for diagnosing blepharitis, hereinafter second method, in a subject which comprises:
(i) determining the level of one or more proteins selected from the group of Lipophilin A, Cystatin S Cystatin N and S100A6 in a sample from a subject; and
(ii) comparing the level of said protein or proteins with the level of said protein or proteins in a reference sample,
wherein if the subject has blepharitis, the level of at least one of S100A6 and Cystatin N proteins is elevated with respect to its level in the reference sample and/or the level of at least one of the Cystatin S and Lipophilin A proteins is reduced with respect to its level in the reference sample.

It is also disclosed that the second method additionally comprises determining the level of one or more proteins selected from the group of Peroxiredoxin 5, S100A4, PLAA, GSTP1, Annexin 11, Annexin 1, Galectin 7, GSTP1, Serpin B13, Reticulocalbin 1, Superoxide dismutase, SMAD3, JNK, GSTM2, Cystatin S, GSTA4 and SSB.

It is also disclosed that the S100A6 protein can individually serve as a marker for the diagnosis of blepharitis in a tear sample from a subject. In subjects with blepharitis, it is observed that the level of the S100A6 protein is elevated with respect to the level of said protein in a reference sample. It is also disclosed that if the protein concentration level is higher than 115 pg/µg and lower than 182 pg/µg with respect to a reference sample, the subject suffers from blepharitis.

It is also disclosed that the CST4 (Cystatin 4 or Cystatin S) can individually serve as a marker for the diagnosis of blepharitis in a tear sample from a subject. In subjects with blepharitis, it is observed that the level of the CST4 protein is reduced with respect to the level of said protein in a reference sample. Preferably, if the subject suffers from blepharitis, the CST4 protein concentration is higher than 98.5 pg/µg but lower than 118 pg/µg.

Also disclosed is that the CST1 (Cystatin 1 or Cystatin N) can individually serve as a marker for the diagnosis of blepharitis in a tear sample from a subject. Preferably, the subject suffers from blepharitis if the concentration of CST1 is higher than 12.4 pg/µg, with respect to a reference sample.

It is also disclosed that the Galectin 7 can individually serve as a marker for the diagnosis of blepharitis in a tear sample from a subject. The subject suffers from blepharitis if the Galectin 7 protein concentration is reduced with respect to the said concentration in the reference sample. Preferably, the subject suffers from blepharitis if the Galectin 7 concentration is lower than 190 pg/µg, with respect to the reference sample.

It is also disclosed that the markers useful for diagnosing blepharitis are: S100A6, CST1, CST4, ANXA1, ANXA11 and Lipophilin A. Thus, when the expression level of one or more of these ANXA1, ANXA11, S100A6 and CST1 is elevated with respect to its level in the reference sample and/or when the expression level of one or more of Lipophilin A and CST4 proteins is reduced with respect to its level in the reference sample, it is indicative of the subject having blepharitis.

Each of these markers can individually serve as a marker for diagnosis of blepharitis. However, combinations of these markers are also possible. Although not forming part of the invention, useful combinations of these markers include the following combinations of 2, 3, 4 and 5 markers:

| | | |
|---|---|---|
| S100A6, CST1 | S100A6, CST1, CST4 | S100A6, CST1, CST4, ANXA11 |
| S100A6, CST4 | S100A6, CST1, ANXA11 | S100A6, CST1, CST4, ANXA1 |
| S100A6, ANXA11 | S100A6, CST1, ANXA11 | S100A6, CST1, CST4, LipophilinA |
| S100A6, ANXA1 | S100A6, CST1, LipophilinA | S100A6, CST1, ANXA11, ANXA1 |
| S100A6, LipophilinA | S100A6, CST4, ANXA11 | S100A6, CST1, ANXA11, LipophilinA |
| CST1, CST4 | S100A6, CST4, ANXA1 | S100A6, CST1, ANXA1, LipophilinA |
| CST1, ANXA11 | S100A6, CST4, LipophilinA | S100A6, CST4, ANXA11, ANXA1 |
| CST1, ANXA1 | S100A6, ANXA11, ANXA1 | S100A6, CST4, ANXA11, LipophilinA |
| CST1, LipophilinA | S100A6, ANXA11, LipophilinA | S100A6, CST4, ANXA1, LipophilinA |
| CST4, ANXA11 | S100A6, ANXA1, LipophilinA | S100A6, ANXA11, ANXA1, LipophilinA |
| CST4, ANXA1 | CST1, CST4, ANXA11 | CST1, CST4, ANXA11, ANXA1 |
| CST4, LipophilinA | CST1, CST4, ANXA1 | CST1, CST4, ANXA11, LipophilinA |
| ANXA11, ANXA1 | CST1, CST4, LipophilinA | CST1, CST4, ANXA1, LipophilinA |
| ANXA11, LipophilinA | CST1, ANXA11, ANXA1 | CST1, ANXA11, ANXA1, LipophilinA |
| ANXA1, LipophilinA | CST1, ANXA11, LipophilinA | CST4, ANXA11, ANXA1, LipophilinA |
| S100A6, CST1, ANXA11, ANXA1, LipophilinA | CST1, ANXA1, LipophilinA | S100A6, CST1, CST4, ANXA11, ANXA1 |
| S100A6, CST4, ANXA11, ANXA1, LipophilinA | CST4, ANXA11, ANXA1 | S100A6, CST1, CST4, ANXA11, LipophilinA |
| CST1, CST4, ANXA11, ANXA1, LipophilinA | CST4, ANXA11, LipophilinA | S100A6, CST1, CST4, ANXA1, LipophilinA |
| ANXA11, ANXA1, LipophilinA | CST4, ANXA1, LipophilinA | |

It is also disclosed an *in vitro* method for differentiating between dry eye and blepharitis, hereinafter third method, in a subject which comprises:
(i) determining the level of one or more proteins selected from Galectin 7, Cystatin S, Cystatin N and S100A6 in a sample from a subject; and
(ii) comparing the level of said protein or proteins with the level of said protein/s in a reference sample,
wherein if the subject suffers from blepharitis, the Galectin 7 concentration is lower than 190 pg/µg, the S100A6 concentration is higher than 115 pg/µg and lower than 182 pg/µg, the Cystatin S protein concentration is higher than 98.5 pg/µg but lower than 118 pg/µg and/or Cystatin N protein level is increased with respect to the concentration in a reference sample.

It is also disclosed that the third method additionally comprises determining the level of one or more proteins selected from the group of Serpin B13, Reticulocalbin 1, Annexin 1, Annexin 11, S100A4, Superoxide dismutase, SMAD3, JNK, GSTM2, GSTA4, Lipophilin A, Peroxiredoxin 5 and SSB is additionally determined.

It is also disclosed that the S100A6 protein can individually serve as a marker for differentiating between dry eye and blepharitis. In subjects with blepharitis, it is observed that the level of the S100A6 protein is elevated with respect to the level of said protein in a reference sample. It is also disclosed that, if the protein concentration level is higher than 115 pg/µg and lower than 182 pg/µg, the subject suffers from blepharitis. It is also disclosed that, if the protein concentration level is higher 182 pg/µg, the subject suffers from dry eye.

It is also disclosed that the CST4 (Cystatin 4 or Cystatin S) can individually serve as a marker for differentiating between dry eye and blepharitis. In subjects with blepharitis, it is observed that the level of the CST4 protein is reduced with respect to the level of said protein in a reference sample. Preferably, if the subject suffers from blepharitis, the CST4 protein concentration is lower than 118 pg/µg but higher than 98.5 pg/µg. It is also disclosed that if the protein concentration level is lower than 98.5 pg/µg, the subject suffers from dry eye.

In a particular embodiment, the CST1 (Cystatin 1 or Cystatin N) can individually serve as a marker for differentiating between dry eye and blepharitis. Preferably, the subject suffers from blepharitis if the concentration of CST1 is increased with respect to the concentration in a reference sample, preferably higher than 12.4 pg/µg. It is also disclosed that, if the subject suffers from dry eye, the concentration of CST1 is reduced with respect to the concentration in a reference sample, preferably lower than 7 pg/µg.

It is also disclosed that the Galectin 7 can individually serve as a marker for differentiating between dry eye and blepharitis. The subject suffers from blepharitis if the Galectin 7 protein concentration is reduced with respect to said concentration in the reference sample. Preferably, the subject suffers from blepharitis if the Galectin 7 concentration is lower than 190 pg/µg and the subject suffers from dry eye if the Galectin 7 concentration is lower than 257 pg/µg, but higher than 190 pg/µg.

It is also disclosed an *in vitro* method for diagnosing dry eye and/or blepharitis, hereinafter fourth method, in a subject which comprises:
(i) determining the level of one or more proteins selected from the group of Lipophilin A, Peroxiredoxin 5, Cystatin S, Cystatin N, PLAA, S100A4, S100A6, GSTP1, Annexin 1, Annexin 11, JNK and Galectin 7 in a sample from a subject; and
(ii) comparing the level of said protein or proteins with the level of said protein or proteins in a reference sample,
wherein if the subject has dry eye or blepharitis, the level of at least one of the S100A4, S100A6, PLAA, GSTP1, JNK, Peroxiredoxin 5, Annexin 11 and Annexin 1 proteins is elevated with respect to its level in the reference sample and/or the level of at least one of the Cystatin S, Cystatin N, Galectin 7 and Lipophilin A proteins is reduced with respect to its level in the reference sample.

It is also disclosed that the subject may suffer from both dry eye and blepharitis pathologies.

It is also disclosed an *in vitro* method for diagnosing blepharitis or dry eye in a tear sample, hereinafter fifth method, from a subject which comprises:
(i) determining the level of Galectin 7 in said sample; and
(ii) comparing the level of said protein with the level of the protein in a reference sample,
wherein if the subject has dry eye, the level of Galectin 7 is reduced with respect to its level in a reference sample.

The Galectin 7 protein can individually serve as a marker for the diagnosis of blepharitis or dry eye in a tear sample from a subject. In both cases, in subjects with blepharitis and in subjects with dry eye, it is observed that the level of the Galectin 7 protein is reduced with respect to the level of Galectin 7 in a reference sample. Therefore, the fifth method is based on the determination of the level of said protein in a tear sample and said level is compared with the level of said protein in a reference sample, allowing the differentiation of subjects with blepharitis or dry eyes from healthy subjects, wherein if the level of Galectin 7 is reduced with respect to the level of said protein in a reference sample, it indicates that the subject has blepharitis or dry eye. For the determination of the level of the Galectin 7 protein and the details of the method, reference is made to the methods described for the rest of the methods throughout the specification.

It is also disclosed that the sample from which the level of proteins is determined for the diagnosis of the pathology, in the method of the invention, is a tear sample from a subject.

The method of the invention is based on the detection and quantification of the proteins in an individual manner or combinations of said proteins, i.e., one, two, three, four, five, six, seven, eight or nine of the aforementioned proteins or combinations of two, of three, of four, of five, of six, of seven or of eight proteins and even the nine proteins. Likewise, the determination of the level of other proteins which are involved in dry eye pathology, also individually or combined, can additionally be included in said method of the invention.

As used in the present invention, the term "subject" relates to any mammalian animal and includes but is not restricted to domestic animals, farm animals, primates and humans, such as human beings, non-human primates, cows, horses, pigs, sheep, dogs, cats or rodents.

The term "marker" will be used indistinctly throughout the invention together with the term "protein".

As used herein, the term "sample" relates to any biological sample which can be obtained from the eye of a subject, such as conjunctiva, tears, cornea, aqueous humor, etc. Preferably, for putting the methods of the invention into practice, said sample is a tear sample from the subject. The tear sample can be obtained by conventional methods known by the person skilled in the art, for example, by means of an absorbent paper, a capillary or an eye spear. The sample can be obtained from subjects previously diagnosed, or not diagnosed with said pathologies, or also from a subject undergoing treatment, or who has been previously treated for one of said pathologies.

Methods for diagnosis comprising the determination of the level of one or more proteins in a sample from a subject are disclosed.

Methods for diagnosis comprising the determination of the level of one or more proteins in a sample from a subject are also disclosed. Said proteins correspond to the following:

### Lipophilin A

This protein, also referred to as "Secretoglobin family 1D member 1" (SG1D1), the accession number of which in the UniProt database on 27 July 2010 is P30044 (SEQ ID NO: 18), is found forming a heterodimer with Lipophilin C. It is expressed in lacrimal glands, thymus, kidneys, testicles, ovaries and salivary glands. It is known that it can bind to androgens and other steroids and to estramustine, in addition to being a chemotherapeutic agent used in prostate cancer. It can also be found under transcriptional regulation of steroid hormones.

### Peroxiredoxin 5

The Peroxiredoxin 5 (PRDX5) protein reduces hydrogen peroxide and alkyl hydroperoxides and is involved in intracellular redox signaling, in inflammatory response and in cell response to reactive oxygen species. Its accession number in the UniProt database on 27 July 2010 is P30044 (SEQ ID NO: 19).

### Cystatin S

Also referred to as Cystatin 4 and CST4, it is a protein which is secreted and strongly inhibits papain and ficin, as well as partially stem bromelain and bovine cathepsin C. It is found in saliva, tears, urine and seminal fluid. Its accession number in the UniProt database on 27 July 2010 is P01036 (SEQ ID NO: 1).

### Cystatin N

This protein, also called Cystatin SN, Cystatin 1 and CST1, the accession number of which in the UniProt database on 27 July 2010 is P01037 (SEQ ID NO: 2), is found in saliva, tears, urine and seminal fluid. It is a very strong inhibitor of papain and of dipeptidyl peptidase I and to a lesser extent of ficin.

### S100A6

It is a calcium-binding protein, the accession number of which in the UniProt database on 27 July 2010 is P06703 (SEQ ID NO: 3). It can interact with other proteins and has an indirect role in the reorganization of the actin cytoskeleton and in cell motility. Annexin 11 is among the proteins with which it interacts.

### S100A4

It is a calcium-binding protein, the accession number of which in the UniProt database on 27 July 2010 is P06702 (SEQ ID NO: 4). It is located in the cytoplasm and/or nucleus of many cell types and is involved in the regulation of the cell cycle progression and in cell differentiation. Specifically, this protein can participate in motility, invasion and polymerization of tubulin, whereas its deficiency has been related to tumor metastasis.

### Glutathione S-transferase P

It is also called GSTP1. It is a homodimer, the accession number of which in the UniProt database on 27 July 2010 is P09211 (SEQ ID NO: 5). Its function is the conjugation of reduced glutathione to a large number of both exogenous and endogenous hydrophobic electrophiles.

### Annexin 1

Annexin 1 (ANX a1) is a calcium/phospholipid-binding protein promoting the fusion of membranes and is involved in exocytosis. This protein, the accession number of which in the UniProt database on 27 July 2010 is P04083 (SEQ ID NO: 7), regulates the activity of phospholipase A2 and is believed to bind between 2 and 4 calcium ions with high affinity.

### Galectin 7

It is believed that Galectin 7 (LGALS7) may be involved in cell-cell and/or cell/matrix interactions, necessary for the control of cell growth. This protein, the accession number of which in the UniProt database on 27 July 2010 is P47929 (SEQ ID NO: 6), is induced by p53.

### Annexin 11

Annexin 11 or Annexin A11 (ANX a11) binds specifically to calcyclin, in a calcium-dependent manner. This protein, the accession number of which in the UniProt database on 27 July 2010 is P50995 (SEQ ID NO: 17), interacts with the S100A6 protein. Anti-ANXa11 antibodies have been found in the serum of patients with some autoimmune diseases, such as rheumatoid arthritis, systemic lupus erythematosus or Sjögren's syndrome.

### Serpin B13

It is believed that the Serpin B13 protein may be involved in keratinocyte proliferation or differentiation. This protein, the accession number of which in the UniProt database on 27 July 2010 is Q9UIV8 (SEQ ID NO: 8), is specifically expressed in the skin.

### Reticulocalbin 1

This protein, also referred to as RCN1 and the accession number of which in the UniProt database on 27 July 2010 is Q15293 (SEQ ID NO: 9), has four binding sites for calcium, although only two of them are active. It is capable of regulating the calcium-dependent activities in the lumen of the endoplasmic reticulum or post-endoplasmic reticulum compartment.

### Superoxide dismutase

Superoxide dismutase (SOD2) destroys free radicals which are normally produced inside the cells and which are toxic for said cells. Genetic variations of SOD2 (the accession number of which in the UniProt database on 27 July 2010 is P04179 (SEQ ID NO: 10)) have been associated with the susceptibility of suffering from diabetic nephropathy.

### SMAD3

It is thus referred to because of its name "Mothers against decapentaplegic homolog 3". Its accession number in the UniProt database on 27 July 2010 is P84022 (SEQ ID NO: 11). It is a transcriptional modulator activated by TGF-beta and by Activin Receptor-Like Kinases Type I. Defects in said protein may be the cause of colorectal cancer.

### JNK

It is also referred to as "Stress-activated protein kinase" or MAPK8 and the accession number of which in the UniProt database on 27 July 2010 is P45983 (SEQ ID NO: 12). It responds to activation by environmental stress and pro-inflammatory cytokines, phosphorylating a large number of transcription factors, first of all AP-1 components, such as JUN, JDP2 and ATF2 and it then regulates the transcriptional activity of AP-1. It has three isoforms: 1, 2, and 3 and each one has different binding patterns, although the phosphorylation efficiency coincides in all the isoforms.

### PLAA

It is also called "phospholipase A2 activator protein" and the accession number of which in the UniProt database on 27 July 2010 is Q9Y263 (SEQ ID NO: 13), it has an important role in the regulation of specific inflammatory pathological processes, regulating the activity of phospholipase A2.

### GSTM2

The GSTM2 protein is also referred to as "Gluthatione S-transferase Mu2" and its accession number in the UniProt database on 27 July 2010 is P28161 (SEQ ID NO: 14). It is capable of conjugating reduced glutathione to a large number of exogenous and endogenous hydrophobic electrophiles.

### GSTA4

The GSTA4 protein is also referred to as "Glutathione S-transferase A4" and its accession number in the UniProt database on 27 July 2010 is 015217 (SEQ ID NO: 15). Like the GSTM2 protein, it is capable of conjugating reduced glutathione to a large number of exogenous and endogenous hydrophobic electrophiles.

### SSB

The SSB protein, also called "Lupus La protein" or "SS type B antigen" has an important role in the transcription of RNA polymerase III. It is believed that this protein, the accession number of which in the UniProt database on 27 July 2010 is P05455 (SEQ ID NO: 16), is a transcription termination factor. It acts by binding to the 3' end of the nascent transcripts of RNA polymerase III. It is involved in the metabolic process of the mRNA of histones and in the modification of tRNA.

The aforementioned proteins are identified in a schematized form in the following table, together with their accession number in the Uniprot database on 28 July 2010 and the sequence identifier of the sequence listing (SEQ ID NO).

| **Gene** | **Protein** | **UniProt/ SwissProt** | **SEQ ID NO:** |
|---|---|---|---|
| **CST4** | Cystatin S = Cystatin 4 | P01036 | 1 |
| **CST1** | Cystatin N = Cystatin 1 | P01037 | 2 |
| **S100A6** | S100 calcium-binding protein A6 | P06703 | 3 |
| **S100A4** | S100 calcium-binding protein A4 | P06702 | 4 |
| **GSTP1** | Glutathione S-transferase P | P09211 | 5 |
| **LGALS7** | Galectin 7 | P47929 | 6 |
| **ANX A1** | Annexin 1 | P04083 | 7 |
| **Serpin B13** | Serpin B13 | Q9UIV8 | 8 |
| **RCN 1** | Reticulocalbin 1 | Q15293 | 9 |
| **SOD2** | Superoxide dismutase | P04179 | 10 |
| **SMAD3** | Mothers against decapentaplegic homolog 3 | P84022 | 11 |
| **JNK** | Stress-activated protein kinase = MAPK8 | P45983 | 12 |
| **PLAA** | Phosp. A2 activator protein | Q9Y263 | 13 |
| **GSTM 2** | Glutathione S-transferase Mu 2 | P28161 | 14 |
| **GSTA 4** | Glutathione S-transferase A4 | 015217 | 15 |
| **SSB** | Lupus La protein, SS type B antigen | P05455 | 16 |
| **ANX A11** | Annexin 11 | P50995 | 17 |
| **SG1D1** | Secretoglobin family 1D member 1 (Lipophilin A) | 095968 | 18 |
| **PRDX5** | Peroxiredoxin 5 | P30044 | 19 |

A skilled person in the art will understand that the present invention relates not only to the specific proteins mentioned in the present invention, but also to variants thereof.

### Variants

As is known in the state of the art, the identity between two proteins is determined by comparing the amino acid sequence of a first and a second protein. Variants according to the present invention include amino acid sequences having at least 60%, 65%, 70%, 72%, 74%, 76%, 78%, 80%, 90%, or 95% similarity or identity with the original amino acid sequence. The degree of identity between two proteins is determined by using computational algorithms and methods which are widely known by the persons skilled in the art. The identity between two amino acid sequences will preferably be determined by using the BLASTP algorithm [BLASTManual, Altschul, S., et al., NCBI NLM NIH Bethesda, Md. 20894, Altschul, S., et al., J. Mol. Biol. 215: 403-410 (1990)]. Said functionally equivalent variants will conserve the capacity to be used as markers for the diagnosis of dry eye or blepharitis, as shown in the Examples of the present invention. Likewise, the variants according to the invention can include post-translational modifications, such as glycosylation, acetylation, isoprenylation, myristoylation, proteolytic processing, etc.

In the context of the present invention, the level of a protein is said to be increased when the level of said protein is elevated with respect to the levels of said protein in the control or reference sample. According to the present invention, the levels of protein are considered to be increased with respect to the levels of said proteins in the reference sample when the levels of protein in the sample from the subject are increased by at least 5%, at least 10%, at least 15%, at least 20%, at least 25%, at least 30%, at least 35%, at least 40%, at least 45%, at least 50%, at least 55%, at least 60%, at least 65%, at least 70%, at least 75%, at least 80%: at least 85%, at least 90%, at least 95%, at least 100%, at least 110%, at least 120%, at least 130%, at least 140%, at least 150% or more.

On the other hand, in the context of the present disclosure, the level of a protein is said to be reduced or repressed when the level of said protein is reduced with respect to the levels of the protein in the control or reference sample. According to the present disclosure, the levels of protein are considered to be reduced with respect to the levels of said protein in the reference sample when the levels of protein in the sample from the subject are reduced by at least 5%, at least 10%, at least 15%, at least 20%, at least 25%, at least 30%, at least 35%, at least 40%, at least 45%, at least 50%, at least 55%, at least 60%, at least 65%, at least 70%, at least 75%, at least 80%: at least 85%, at least 90%, at least 95%, at least 100%, at least 110%, at least 120%, at least 130%, at least 140%, at least 150% or more.

Table 1 indicates the level of the proteins/markers which are analyzed in the first method of the invention, i.e., if the level of the protein is elevated (+) with respect to the level of said protein in the reference sample or reduced or repressed (-) with respect to the level of said protein in the reference sample. On the other hand, Table 2 indicates the level of the proteins/markers which are analyzed in the second method of the invention, i.e., if the level of the protein is elevated (+) with respect to the level of said protein in the reference sample or reduced, repressed (-) with respect to the level of said protein in the reference sample, or if the level of the protein is unchanged (=) with respect to the level of said protein in the reference sample. Table 1 and Table 2 also include the number of times (fold) by which the level of the protein is altered with respect to the level of said protein in the reference sample.

**Table 1: Levels of each of the proteins detected in the first method of the invention. The methods based on markers CST4, CST1, S100A4, GSTP1, LGALS7, ANX A1 or SG1D1 do not form part of the invention. The (-) sign indicates that level of said protein is reduced or repressed with respect to the level of the protein in the reference sample, whereas the (+) sign indicates that the level of said protein is increased with respect to the level of the protein in the reference sample.**

| **Gene** | **Protein** | **Dry eye** | **Fold** |
|---|---|---|---|
| **CST4** | Cystatin S = Cystatin 4 | - | - 1.78 |
| **CST1** | Cystatin N = Cystatin 1 | - | - 1.89 |
| **S100A6** | S100 calcium-binding protein A6 | + | 5.39 |
| **S100A4** | S100 calcium-binding protein A4 | + | 3.28 |
| **GSTP1** | Glutathione S-transferase P | + | 5.71 |
| **LGALS7** | Galectin 7 | - | - 6.21 |
| **ANX A1** | Annexin 1 | + | 2.96 |
| **SG1D1** | Lipophilin A (Secretoglobin family 1D member 1) | - | - 2.00 |

**Table 2: Levels of each of the proteins detected in the method for the diagnosis of blepharitis. The (-) sign indicates that the level of said protein is reduced or repressed with respect to the level of the protein in the reference sample, whereas the (+) sign indicates that the level of said protein is increased with respect to the level of the protein in the reference sample. The (=) sign indicates that the level of said protein is at the same level with respect to the level of the protein in the reference sample.**

| **Gene** | **Protein** | **Blepharitis** | **Fold** |
|---|---|---|---|
| **CST4** | Cystatin S = Cystatin 4 | - | - 1.96 |
| **CST1** | Cystatin N = Cystatin 1 | - | - 3.64 |
| **S100A4** | S100 calcium-binding protein A4 | = | = |
| **GSTP1** | Glutathione S-transferase P | + | 1.20 |
| **LGALS7** | Galectin 7 | - | - 3.37 |
| **ANX A1** | Annexin 1 | + | 1.45 |
| **SG1D1** | Lipophilin A (Secretoglobin family 1D member 1) | - | - 2.12 |
| **PRDX5** | Peroxiredoxin 5 | - | - 1.62 |

In the context of the present invention, the term "reference sample" or control sample is understood as the biological sample from a healthy subject which is used to determine the variation of the levels of the proteins used in the present invention. In an embodiment, the level of the protein in the reference sample is obtained from the signal provided by using a sample from a healthy individual, who has neither dry eye syndrome nor blepharitis, nor has undergone processes of eye surgery, allergy, atopy, nor medication with corticoids or antiglaucoma agents.

The determination of the levels of the proteins or markers can be carried out by means of immunological techniques, such as ELISA (Enzyme-Linked Immunosorbent Assay, Western blot, RIA (radioimmunoassay), competitive EIA (competitive enzyme immunoassay), DAS-ELISA (Double Antibody Sandwich ELISA), immunocytochemical and immunohistochemical techniques, techniques based on the use of protein biochips or microarrays including specific antibodies or assays based on colloidal precipitation in formats such as dipsticks. The Western blot technique is based on the detection of proteins previously separated by electrophoresis in a gel, under denaturing conditions and immobilized in a membrane which is subsequently incubated with one or more antibodies specific for the protein and is detected by means of a system, for example, a chemiluminescent or fluorescent system. The immunofluorescence analysis requires the use of a specific antibody labeled with a fluorescent compound. The ELISA technique is based on the use of antigens or antibodies labeled with enzymes, such that the conjugates formed between the target antigen and the labeled antibody results in the formation of enzymatically active complexes. Due to the fact that one of the components (the antigen or the labeled antibody) is immobilized in a support, the antibody-antigen complexes are also immobilized in the support and therefore they can be detected by adding a substrate which is converted by the enzyme into a product which is detectable by spectrophotometry or fluorometry, for example. The proteins are preferably detected by Western blot, ELISA, a protein array or by two-dimensional electrophoresis.

The skilled person in the art will understand that, if the level of the proteins is to be determined by two-dimensional electrophoresis, the gel must be stained for the purpose of detecting said proteins and it must be done such that it is quantitative. Any staining method sensitive enough to detect the proteins/markers of the invention can be used and includes but is not limited to silver staining, Coomassie blue staining, Sypro Ruby staining, fluorescent cyanine staining, such as that described in Mujumdar, R. B. et al., (Cytometry, 1989, 10:11-19 and US5268486), dipyrrometheneboron difluoride staining, such as that described in US4774339, etc. Once the spots showing statistically significant differences between the reference sample and the sample from the patient are identified, the proteins described in the method of the invention are identified according to their molecular weight and their isoelectric point. The identity of the protein spots identified in the two-dimensional gel can be confirmed by cutting the spot from the gel, carrying out an in-gel digestion and subsequent mass spectrometry. The levels of protein in the selected spots can be quantified by using any known technique, such as stained gel densitometry and determination of the volume (intensity x height), differential in-gel electrophoresis (DIGE), wherein the proteins to be separated are labeled with fluorophores as a step prior to electrophoretic fractionation and they are then mixed with an internal standard, representing all the proteins in the sample and which can be used for normalization purposes. Likewise, the regions of the gels in which the proteins are differentially expressed can be cut and the identity of the spot of the protein can be identified by using techniques such as MALDI (MALDI-MS/MS) mass spectrometry, electrospray ionization mass spectrometry (ESI-MS/MS). Alternatively, peptide digestion can be carried out and the peptide profile can be determined by mass spectrometry, Edman degradation sequencing or n N-terminal sequencing.

The final step of the method of the invention comprises comparing the level of the protein or proteins with the level or the amount of said protein-marker detected in the reference samples from control subjects or in previous samples from the same individual.

Additionally, if desired, the determination of the level of proteins in a sample from an individual having the pathology can be incorporated to the method of the invention as a positive control, such that it is verified that the method of the invention is effective.

### Kit of the invention

It is also disclosed a kit useful in the implementation of the methodology described herein. Thus, said kit comprises at least one reagent for the detection of at least one protein selected from the group of Lipophilin A, Peroxiredoxin 5, Cystatin S, Cystatin N, S100A4, S100A6, GSTP1, PLAA, Annexin 1, Annexin 11 and Galectin 7 or a functional variant thereof. In other words, the kit includes a set of reagents for detecting the expression levels of all the proteins or of at least one, two, three, four, five, six, seven, eight or nine of the proteins, or functionally equivalent variants of said proteins. Thus, the kit comprises the reagents for the detection of one or more of the following proteins: Lipophilin A, Peroxiredoxin 5, Cystatin S, Cystatin N, S100A4, S100A6, GSTP1, PLAA, Annexin 1, Annexin 11 and Galectin 7 or functionally equivalent variants thereof.

It is also disclosed a kit comprising the aforementioned reagents and, additionally, reagents for the detection of one or more proteins selected from the group of Serpin B13, Reticulocalbin 1, Superoxide dismutase, SMAD3, JNK, GSTM2, GSTA4 and SSB. The invention relates to a kit comprising at least one reagent for the detection of protein S100A6 or a functional equivalent variant thereof for its use in the in vitro method for diagnosing dry eye in a subject according to the invention, said kit additionally comprising reagents for the detection of one or more proteins selected from the group of Serpin B13, Reticulocalbin 1, Superoxide dismutase, SMAD3, GSTM2,GSTA4 and SSB.

The kit for use of the invention optionally comprises a positive control including a known sample, which has a pattern of levels of proteins which coincides with a sample from a subject having dry eye.

It is also disclosed the kit for use in the *in vitro* diagnosis of blepharitis or for differentiating between dry eye and blepharitis.

The invention is illustrated below based on the following examples which are provided to illustrate but not to limit the scope of the invention.

### EXAMPLES

### MATERIALS AND METHODS

### Tear sampling:

Tear samples were taken from different subjects by using a Merocel sponge (PVA 0525; Oasis, Glendora, CA), without applying any type of anesthesia. The sponges were then left in an Eppendorf tube containing 200 µl of PBS (phosphate buffer) for 1 hour for the purpose of achieving a more efficient elution of the proteins. The protein material was collected by means of centrifugation at 4°C and stored at -80°C until the time of the analysis. The samples were classified according to the inclusion and exclusion criteria mentioned below:
Exclusion Criteria:
   - Eye surgery (2 months ago)
   - History of allergies
   - Atopy
   - Medications with corticoids
   - Chronic medications (Glaucoma)
   - Any medication except artificial tears
   - Contact lenses
General Inclusion Criteria:
   - Age above 18 years
   - Signing the informed consent
Inclusion Criteria by group:
   - Healthy controls:
      a. Absence of dry eyes and/or blepharitis symptoms
      b. Schirmer test >5 mm
      c. Not contact lenses wearers
      d. No atopy
   - Dry eyes:
      a. Dryness or burning sensation symptoms which worsen in dry environments and throughout the day
      b. Schirmer test <5 mm
   - Blepharitis:
      a. Eyelid inflammation
      b. Eye discomfort and episodic reddening symptoms
      c. Vascularization in eyelid edge
      d. Itching
      e. Schirmer test >5 mm
      f. Slit lamp: hyperemia and thickening of the free eyelid edge; meibomian gland dysfunction; instability of the tear film (with fluorescein).

### EXAMPLE 1: Validation of markers by Western blot MATERIALS AND METHODS

### 1D-SDS-PAGE and Western Blot:

Samples were taken from 39 subjects in order to carry out the process for validating markers previously described in the literature (Lipophilin A and Peroxiredoxin 5). Thus, according to the aforementioned inclusion and exclusion criteria, the 39 samples from subjects were divided as follows: 13 individuals of the control group, 12 individuals belonging to the group of patients with dry eyes and 14 individuals belonging to the group of patients with blepharitis. The sample was collected in exactly the same way as in the case of the tear samples obtained in Example 2.

For the validation of the Lipophilin A and Peroxiredoxin 5 markers, a comparative expression study was carried out by means of SDS-PAGE electrophoresis in 15% acrylamide gels followed by immunofluorescent multiplex western blot on low-fluorescence PVDF (Polyvinylidene Fluoride) membranes. To carry out this assay, for the detection of Peroxiredoxin (PRDX5), a primary anti-PRDX5 antibody produced in mice was used and a secondary anti-mouse antibody conjugated with AlexaFluor 633 nm (Invitrogen Dynal AS, Oslo, Norway) was used for its detection. The detection of lipophilin A was performed using goat anti-SG1D1 and a secondary anti-goat antibody conjugated with AlexaFluor 488 nm (Invitrogen Dynal AS, Oslo, Norway) for its detection. The idea of performing this multiplex assay consists of being able to use the same samples for analyzing several markers using two different excitation/emission channels, and leaving a third one for the normalization by means of total protein staining with Sypro Ruby® (Invitrogen Dynal AS, Oslo, Norway). The optimal conditions of antibodies for the detection of the proteins were established, using a 1:1000 ratio for the primary antibodies and a 1:5000 ratio for the secondary antibodies.

The fluorescence volume value (area x fluorescence intensity) of the band corresponding to each of the markers was extracted from each of the Western blots and normalized with respect to the total protein value. The corresponding statistical analyses (ANOVA, Student's t-test, discriminant analysis, dot-plot interactions and linear regression) were subsequently performed.

### RESULTS

In the Western blots, in the case of Peroxiredoxin 5, the appearance of two isoforms, which were referred to as A and B (A being the one with the highest molecular mass), was detected, so each of them were assessed individually and, also, both isoforms were assessed jointly. Figure 1 shows the results of the Western blot for Lipophilin A and for Peroxiredoxin 5, for dry eyes and blepharitis, compared with the controls. Figure 2 depicts the normalized volume values for each group and for each marker. A reduction in the Lipophilin A concentration, both in dry eyes and in blepharitis with respect to the control, can be observed. In the case of Peroxiredoxin 5, both in dry eyes and in blepharitis, the presence of an isoform (PRDX5-A), which was virtually not present in the control group, was observed.

After performing the corresponding ANOVA and Student's t-test statistical analyses, it was possible to verify that both Lipophilin A and Peroxiredoxin 5 were capable of differentiating each of the pathologies with respect to the control with probability values (p-values) lower than 0.05. Lipophilin A presented a statistically significant difference between the control group and the blepharitis group. Isoform A of Peroxiredoxin 5 was capable of differentiating the control group both from dry eyes and from blepharitis, whereas isoform B of Peroxiredoxin 5 was capable of exclusively differentiating between blepharitis and controls, indicating its potential as a differential diagnostic marker for blepharitis. Finally, taking into account both isoforms together, a differentiation of dry eyes versus the blepharitis group was observed, which is a very important piece of data since it is a differential diagnosis between both pathologies with respect to the control. Table 3 shows the probability values associated with these analyses, highlighting in gray those which are significant with a confidence level of 95% (p<0.05). The increase in the PRDX5 protein level, isoform A, was also confirmed by the results shown in the ELISA assays of Example 5.

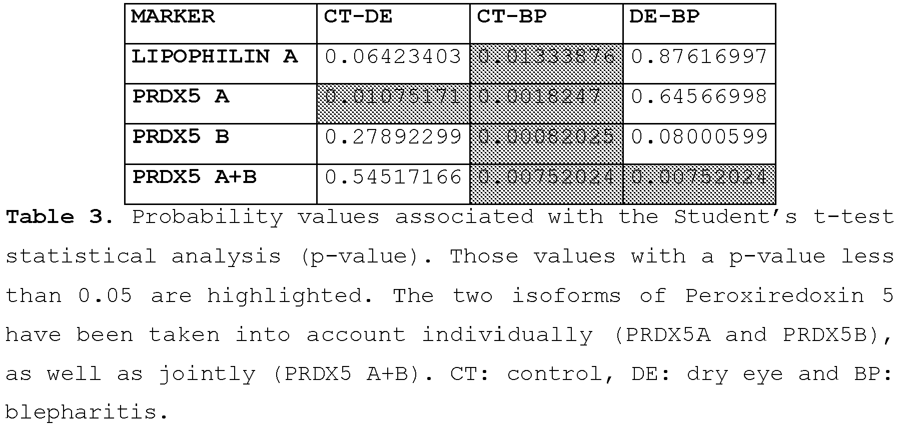

Likewise, when the data were analyzed by means of simple linear regression using logical values for the independent variable (0 and 1), the result obtained allows determining the prediction capacity which is obtained from the data. In other words, if the relationship between the groups is linear, slope b associated with the straight line estimates the "effect" of the independent variable on the dependent variable, and allows analyzing the hypothesis of independence between the two variables. Furthermore, the intensity of the relationship is evaluated with the R² index and predictions can be made from the model. When the logistic regression which is performed is simple it is verified that, despite the analyzed markers being capable of differentiating between the different groups, the percentage of patients the group of which could be predicted in the event of using a single marker would be 85.19% for the comparison of the blepharitis pathology with the control group. However, when the regression analysis is approached taking into account the 2 variables present in the system (Peroxiredoxin 5 and Lipophilin A values), the effectiveness in the prediction increases up to 92.59%, a value which indicates the percentage of correctly classified cases.

The correct classification of the cases has also been approached by means of studying ROC curves and interactive dot plots, as shown in Figure 3, which depicts the normalized volume values obtained for Peroxiredoxin 5 isoform A, and total (PRDX5A and PRDX5B) by means of Western blot. It can be observed that the sensitivity and specificity values in both dry eyes-control and blepharitis-control comparisons are suitable, indicating that they can be suitable markers for both pathologies.

In addition, upon performing discriminant analysis as a multivariate statistical tool and using values of both Lipophilin A and of Peroxiredoxin 5, isoforms A and B, for the analysis, a suitable clustering of all the classes was seen, as shown in Figure 4. Likewise, a perfect clustering of the dots of the control group was observed, except for a single dot. It can also be observed that the rest of the groups are well defined although overlapping one another with the exception of the dry eye group, which forms two independent clusters separated from one another. The two subgroups found within dry eyes will probably be due to the type of the pathology or to the degree of progress of said pathology. However, despite the fact that the control group is well separated from the rest, this analysis also indicates an overlapping between the groups of patients with blepharitis and dry eyes. The classification of the individuals by means of learning techniques indicates that it is possible to differentiate between the three groups in 69.3% of the cases, with a correct classification maximum of 92.9% in the case of blepharitis.

Using this statistical methodology it was possible to determine the minimum number of markers necessary to predict to which group from among the three of the study (dry eyes, blepharitis and controls) any test sample belongs.

### EXAMPLE 2: Identification of markers by two-dimensional electrophoresis MATERIALS AND METHODS

### Samples

A prospective case-controlled study was carried out, in which tear samples were collected from 44 subjects from Hospital Cruces, (Baracaldo, Vizcaya) who were divided as follows: 16 of them belonged to the group of patients with blepharitis, 16 to patients with dry eyes and 12 to control individuals. Diagnosis was based on clinical examinations that include Schirmer's test (with anesthesia), biomicroscopy with careful examination of the lid margin and meibomian glands, fluorescein assisted slit-lamp examination, and subjective symptoms.

In this case, the tear samples were processed to remove IgA and serum albumin by means of immunoaffinity chromatography using a Seppro column (Sigma-Aldrich, St. Louis, MO, United States). Two of the major proteins of tears were thus removed from the samples, leaving other less abundant proteins more accessible, both for the analysis and for their subsequent identification. The flow-through sample fraction (not retained in the column) was precipitated with the CleanUp kit (GE Healthcare, Sunnyvale, CA, United States) for the removal of lipids and salts present in the samples following the protocol described by the manufacturer. After resuspending the pellets resulting from the precipitation in 100 µl of DeStreak (GE Healthcare, Sunnyvale, CA, United States), the samples were left solubilizing for 24 hours and they were quantified by means of a fluorometric technique based on the staining of total protein on paper (EZQ Invitrogen Dynal AS, Oslo, Norway).

One of the problems encountered when working with tears is the small amount of protein content obtained from each subject, due to the reduced volume obtained in the tear samples. The requirements in terms of the total protein load in order to be able to perform the 2D gels in the 17 cm format are a total of 40 µg per gel. In addition to the fact that most of the samples do not meet this requirement, a large part of this protein content is removed by means of immunoaffinity chromatography. As a result, some samples are obtained which do not reach the amount of protein required for the study and therefore these patients cannot be included in the study. For this reason, the samples of the same class were grouped into twos in an equimolar manner, and finally 8 gels of the blepharitis group (16 patients), 6 gels of the control group (12 individuals) and 8 gels of the dry eye group (16 patients) were analyzed.

40 µg of total protein of each of the pools of the samples were taken to a volume of 300 µl using DeStreak® (GE Healthcare, Sunnyvale, CA, United States) and supplementing with DTT (dithiothreitol) to a final concentration of 20 mM. All the samples were then focused in 17 cm strips with a pH range of 4-9, using the same number of Vxh and in a Protean IEF Cell device (BioRad, Malvern, Pennsylvania, USA), following a protocol which consisted of 2 stages: after 7 hours of passive hydration have elapsed, an active hydration was carried out at 50 V for 8 hours; the voltage was then taken to 10,000 V in an exponential gradient and the strips were left "focusing" for 60,000 Vxh in order to be used immediately afterwards in the second dimension. The already focused strips were equilibrated with equilibration buffer I (6 M Urea, 2% SDS, 29.3% glycerol, 2% Dithiothreitol (DTT)) for 15 minutes followed by another 15 minutes of equilibration with equilibration buffer II (6 M Urea, 2% SDS, 29.3% glycerol, 2.5% Iodoacetamide (IA)). Once reduced and carboxymethylated, the strips were resolved according to their molecular mass by means of SDS-PAGE electrophoresis under denaturing conditions in large 15% polyacrylamide gels (20x26 cm) using the DALT-SIX electrophoresis system (GE Healthcare, Sunnyvale, CA, United States) according to the standard protocol. The gels were viewed using a VersaDoc image analyzer (BioRad, Malvern, Pennsylvania, USA) after staining the gels with Sypro Ruby® (Invitrogen Dynal AS, Oslo, Norway) following the indications of the manufacturer.

### Image acquisition and data analysis

Once gels were stained with Sypro Ruby, fluorescence reactive proteins were visualized using a VersaDoc Imager (BioRad, Malvern, Pennsylvania, USA) following the manufacturer's instructions. Image analysis was performed with Progenesis SameSpots Software, version 4.0 (NonLinear Dynamics Limited, Newcastle, UK). Significant differences between groups were determined by the significance analysis of microarrays (SAM) method (Tusher VG et al. Proceedings of the National Academy of Sciences of the United States of America 2001;98:5116-21) using normalized spot volumes obtained from Progenesis SameSpots Software. The SAM uses the concepts of the false discovery rate (FDR) (Hochberg Y et al. J Roy Statist Soc Ser B (Methodological) 1995;57:289-300) and the q value (Storey J. J Roy Statist Soc B 2002;64:479-98). The FDR differs from more conventional corrections for multiple comparisons in that instead of controlling for false positives, it controls for the expected ratio of false positives among significantly expressed proteins. The q value is a posterior Bayesian p value, and it refers to the minimum FDR at which a test is deemed to show a statistically significant difference. In this analysis, the FDR was set at 1%, with a significant difference in protein abundance defined as one yielding a q value less than 0.01. The clustering of the samples once the data were filtered was performed by means of data exploration techniques such as the principal component analysis (PCA). Ascending hierarchical cluster analyses were also used to check the proximity existing between the samples as well as between the variables, using for that purpose the *Wards Linkage* method and the Euclidean distance measurement unit by means of the Orange Canvas statistical package (http://orange.biolab.si/).

### MALDI-TOF analyses

Protein spots of interest were cut from 2-D SYPRO Ruby stained electrophoresis gels and subjected to in-gel trypsin digestion according to Shevchenko (Nature protocols 2006;1:2856-60) with minor modifications. The gel pieces were swollen in a digestion buffer containing 50 mM NH₄HCO₃ and 12.5 ng/uL trypsin (Roche Diagnostics, recombinant, proteomics grade trypsin, Penzberg, Germany) on an ice bath. After 30 minutes, the supernatant was removed and discarded. Then, 20 µL of 50 mM NH₄HCO₃ were added to the gel piece and the digestion was allowed to proceed at 37°C overnight. The supernatant was transferred to an empty eppendorf tube and basic and acidic peptide extraction was performed on the gel pieces. Supernatants of each sample were pooled and dried by vacuum centrifugation. Prior to mass spectrometry (MS) analysis, pellets were resuspended in 10 µL 0.1% trifluoroacetic acid (TFA). Recovered peptides were purified prior to MALDI analysis by custom made nano-columns as described by Gobom 10 with some modifications including a column consisting of 100-300 nL of POROS R2 material (PerSeptive Viosystems, Framingham, MA). The column was equilibrated with 0.1% TFA and the bound peptides subsequently eluted directly onto the MALDI target with 0.5 µL CHCA solution (20 µg/µL in ACN, 0.1% TFA, 70:30, vol/vol). Peptide mass fingerprinting was performed on a Bruker Ultraflex TOF/TOF mass spectrometer (Bruker-Daltonics, Bremen, Germany). Positively charged ions were analyzed in reflector mode, using delayed extraction. The spectra were obtained by randomly scanning the sample surface. Typically 600-800 spectra were averaged to improve the signal to noise ratio. Spectra were externally calibrated resulting in a mass accuracy of < 50 ppm. Protein identification was performed by searching in a non-redundant protein database (NCBI) using the Mascot search engine (http://www.matrixscience.com).

### RESULTS

Figure 5 shows an image of the proteome of each of the study groups, in which the high level of resolution which was reached in the tear analysis (two-dimensional gels) can be observed. As a result of these analyses, a tear protein map formed by 130 resolved spots in the pH range between 4 and 9 was obtained (Figure 5D). Figure 5A-C shows a representative image of the proteome of each of the study groups, where a differentiation of the protein profile according to the type of pathology can be observed. In order to be able to carry out this process, a filtering of the data with a critical q-value less than 0.01, a statistical power value for each of the spots greater than 0.995, was performed. Thus, the proteins which had greater expression changes and which therefore caused a more effective separation of the three groups were identified by means of MALDI-TOF mass spectrometry.

The differential expression analysis of the resulting gels by means of the Progenesis SameSpots computer program shows that although there are variations in the expression levels throughout the entire gel, it can be emphasized that the most significant changes occur at low molecular mass. As a result, a series of spots differentially expressed between the different groups was found, as shown in Figure 6. Besides, the differences in the expression levels found in some of these proteins can be also observed in the relief map shown in Figure 7.

The multivariate statistical analysis (principal component analysis - PCA) indicated in Figure 8 performed from the raw data of expression of those spots which are statistically significant assured a perfect separation between the different groups. This perfect separation between groups indicates that the selected spots could be potential biomarkers since they are capable of differentiating the groups completely, therefore said spots have been identified by means of MALDI-TOF/TOF mass spectrometry. The results obtained, associated with the changes of expression and ANOVA values, are shown in Table 4.

**Table 4: Results obtained associated with changes of expression and ANOVA values of several markers with respect to their different expression in the different groups of patients (DE) dry eyes, (BP) blepharitis and (CT) control group. The normalized volume values of the proteins with greater variation between the groups and statistically significant values are shown. The "Fold" value (or number of times) has been calculated taking the highest value between dry eyes or blepharitis in the case of overexpression and dividing it by the control group value, or, in the case of repression, with a negative symbol, the control value divided by the lowest value between dry eye or blepharitis. [The values indicated with * correspond to the highest values, and the values indicated with ** to the lowest values].**

| | | | **Normalized volume** | | |
|---|---|---|---|---|---|
| **Anova (p)** | **Fold** | **Protein** | **DE** | **BP** | **CT** |
| 9.74E-05 | 5.71 | Glutathione S-transferase P | **4.57E+05*** | 9.61E+04 | **8.00E+04**** |
| 6.03E-04 | 3.28 | S100-A4 Protein | **5.87E+05*** | **1.71E+05**** | 1.79E+05 |
| 0.001 | - 3.7 | Cystatin-N | 2.63E+05 | **1.36E+05**** | **4.95E+05*** |
| 0.002 | 5.39 | S100-A6 Protein (isoform 1) | **2.19E+06*** | **2.66E+05**** | 4.06E+05 |
| 0.007 | 3 | Annexin A1 | **2**.**26E**+**05*** | 1.11E+05 | **7.64E+04**** |
| 0.009 | 4.04 | S100-A6 Protein (isoform 2) | **8.05E+05*** | **1**.**49E**+**05**** | 1.99E+05 |
| 0.023 | - 1.96 | Cystatin-S | 1.29E+06 | **1**.**14E**+**06** | **2**.**23E**+**06** |
| 0.011 | - 6.2 | Galectin-7 | **5**.**54E**+**04** | 1.02E+05 | **3**.**44E**+**05** |

Once the proteins which, according to the proteomics studies, are capable of differentiating better between the different groups (Annexin A1, Cystatin S, Cystatin N, Glutathione S-transferase P, S100A4 and S100A6) have been identified by means of mass spectrometry, the inventors performed a expression and functional networks analysis between identified proteins by means of using bioinformatics tools such as Greedy Thick Thinning method, and a cluster of a total of 19 candidate proteins was selected for the subsequent validation studies.

These validation analyses include the candidate markers resulting from the differential expression analysis, together with those proteins which are directly or indirectly related through expression and functional networks, as well as those markers described in the literature for the pathologies analyzed in this study.

The expression analyses indicated that there are at least two types of protein profiles according to the expression levels found for those proteins. On one hand, there is a group of six proteins having an increase of expression between the dry eye group with respect to the control or blepharitis group, such as the proteins S100A4, S100A6, S100A8, S100A9, GSTP1 and ANXA1 which have folds of 3.85, 8.54, 3.25, 6.87, 6.92 and 4.16 respectively (Table 5). In the same way, it was also possible to verify the presence of another panel of proteins the expression levels of which were considerably reduced in the pathological groups with respect to the control group, as in the case of the proteins LCN1, AZGP1, PIP, CST1, CST4, LEG7, ACTB and LTF, which have expression change values of 11.47, 2.77, 3.22, 5.15, 4.58, 7.39, 1.85 and 14.53 fold respectively (Table 5). The decrease of CST1 and CST4 is particularly related to the BL group, and the rest of proteins in the panel with DE.

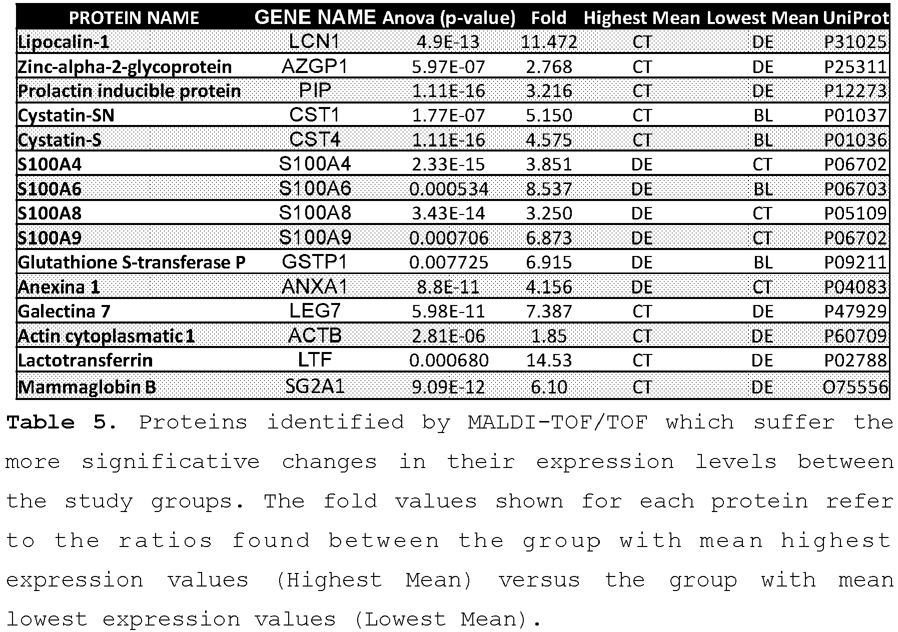

### EXAMPLE 3: Biological functionality study

The principal component analysis (PCA) performed from the raw data of expression of those spots which are statistically significant assures a perfect separation between the different groups, as can be seen in Figure 8. This means that the selected spots are potential biomarkers, so they have been identified by means of MALDI-TOF/TOF mass spectrometry. Once the marker proteins have been identified by mass spectrometry, a study of their biological functionality was performed. A functional analysis with the bioinformatics tool "DAVID" (Database for **A**nnotation, **V**isualization and **I**ntegrated **D**iscovery) (http://david.abcc.ncifcrf.gov/home.jsp) was carried out with all the proteins resulting from the expression network generated, for the purpose of verifying the biological functions having a greater correlation with the expression changes found in the study and therefore with a greater involvement in the pathologies studied.

This process of generating expression networks is in turn a tool which can reveal new molecules which could be considered as putative markers since their interconnection with the most significant proteins of this study indicates that the expression changes found could be the result of deregulations occurring in other network components. Thus, the inventors verified that a high percentage of the candidates to new markers were compromised in functions related to inflammation, immune response and to oxidative stress, as illustrated in Figure 9, biological processes which are closely related to the dry eye and blepharitis pathologies.

Once the expression and functional networks formed by the 7 previously identified markers have been analyzed, and observing that the biological processes most involved were the inflammatory and oxidative stress processes, 10 new markers related to these processes were incorporated to the group of markers for validation, these new markers being: PLAA, GSTM 2, GSTA 4, SSB, Annexin 11, Serpin B3, RCN1, SOD2, SMAD3 and JNK.

Table 6 shows a list with the names of the candidates to markers for the dry eyes and blepharitis pathologies, classified into three groups (Groups 1, 2 and 3), according to way in which they are obtained. Group 1 includes the markers obtained from the differential expression studies by means of 2-D SDS-PAGE, Group 2 includes those obtained by expression network analysis and functional enrichment analysis and Group 3 includes those previously reported in the literature and validated by Western blot.

**Table 6: List of markers for dry eye and blepharitis pathologies**

| **Gene** | **Protein** | **UniProt/ SwissProt** |
|---|---|---|
| **GROUP 1** | | |
| **CST4** | Cystatin S = Cystatin 4 | P01036 |
| **CST1** | Cystatin N = Cystatin 1 | P01037 |
| **S100A6** | S100 calcium-binding protein A6 | P06703 |
| **S100A4** | S100 calcium-binding protein A4 | P06702 |
| **GSTP1** | Glutathione S-transferase P | P09211 |
| **LGALS7** | Galectin 7 | P47929 |
| **ANX A1** | Annexin 1 | P04083 |

| **GROUP 2** | | |
|---|---|---|
| **Serpin B13** | Serpin B13 | Q9UIV8 |
| **RCN 1** | Reticulocalbin 1 | Q15293 |
| **SOD2** | Superoxide dismutase | P04179 |
| **SMAD3** | Mothers against decapentaplegic homolog 3 | P84022 |
| **JNK** | Stress-activated protein kinase= MAPK8 | P45983 |
| **PLAA** | Phosp. A2 activator protein | Q9Y263 |
| **GSTM 2** | Glutathione S-transferase Mu 2 | P28161 |
| **GSTA 4** | Glutathione S-transferase A4 | 015217 |
| **SSB** | Lupus La protein, SS type B antigen | P05455 |
| **ANX A11** | Annexin 11 | P50995 |

| | **GROUP 3** | |
|---|---|---|
| **SG1D1** | Secretoglobin family 1D member 1 (Lipophilin A) | 095968 |
| **PRDX5** | Peroxiredoxin 5 | P30044 |

### Expression networks and functional analysis

The list of candidates for molecular markers has thus been extended to a total of 17, adding to the 14 found in this study by means of 2D analysis (Table 5), the new putative molecular markers proposed from the expression networks and shown in Table 7.

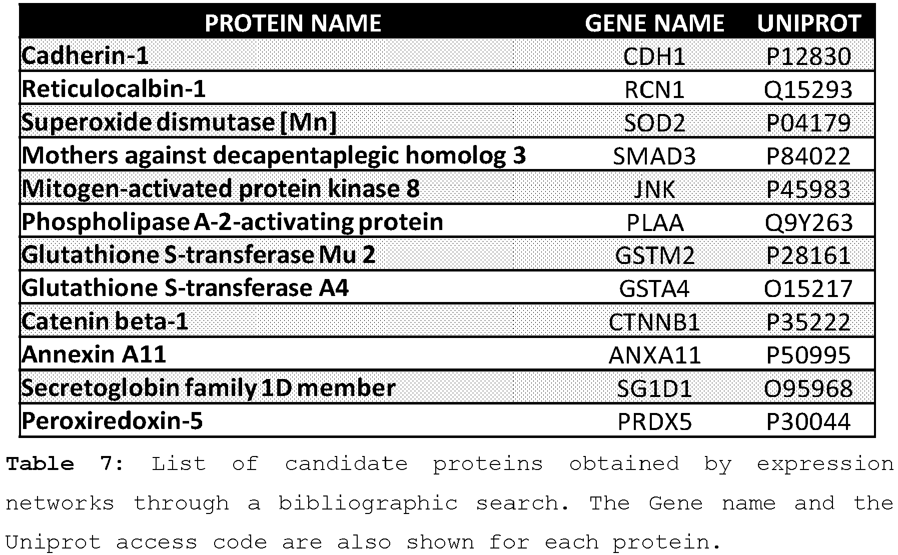

### EXAMPLE 4: Validation of GAL-7 by means of sandwich ELISA

Validation studies for the GAL-7 protein have been performed by means of Sandwich type ELISA, adding 5 µg of total protein in each of the cases. The quantification of both ELISA systems is based on an indirect detection of the antigens. Biotinylated primary anti-GAL-7 antibodies were added first. A second incubation was subsequently performed with horseradish peroxidase (HRP)-conjugated streptavidin and finally tetramethylbenzidine (TMB) for the development and 1 M sulfuric acid, for the purpose of stopping the reaction. The signal of the wells was detected by means of measuring the absorbance at 450 nm. Both ELISA kits incorporated a calibration line in the range between 3333 pg/ml and 13.72 pg/ml with a minimum sensitivity of 20 pg/ml. Once the results of both assays were obtained, it was verified that the variations between the groups in the case of GAL-7 were significant. The statistical analysis performed for the concentration values obtained for this protein indicates, for Dry Eyes versus the reference sample, sensitivity and specificity values for this marker of 100% and 91.7 respectively, and an area under the ROC curve (AUC) of 0.988, as shown in Figure 10, the quantification of this marker indicating an excellent precision as a diagnostic methodology. Likewise, Figure 11 indicates, in the case of blepharitis versus the reference sample, that the critical point of Galectin 7 concentration is 1790 pg/ml, which indicates that 91.7% specificity and 75% sensitivity are achieved below said value.

### EXAMPLE 5: Validation of markers by ELISA assays MATERIALS AND METHODS

### Patients

A second prospective case-controlled study was carried out, in which 103 patients were enrolled. Patients and normal subjects were recruited from the Cornea and Ocular Surface Unit at Instituto Clinico Quirúrgico of Oftalmologia, (Bilbao, Vizcaya), Instituto Oftalmológico Fernandez Vega, (Oviedo, Asturias), and Hospital of Valladolid (Valladolid, Castilla León). Diagnosis was based on clinical examinations that include Schirmer's test (with anesthesia), biomicroscopy with careful examination of the lid margin and meibomian glands, fluorescein assisted slit-lamp examination, and subjective symptoms. Each patient was asked for subjective symptoms such as burning, itching, foreign body sensation, dryness, photophobia, presence of sleeps or scales on the eyelids, stuck eyes, between others.

Patients were classified as having dry eye if they had dry eye symptoms and abnormalities of test dynamics determined by Schirmer (≤5 mm/5 min). Patients were diagnosed as blepharitis if they presented eyelids inflammation, symptoms and Schirmer (≥5 mm/5 min). The most symptomatic eye from each patient was analyzed. In the control group healthy subjects were recruited who were not suffering from any ocular disease (no allergic or atopic history).

The exclusion criteria included the presence or history of any systemic or ocular disorder or condition (including ocular surgery, trauma and disease) that could possibly interfere with the interpretation of the results. Current or recent use of ophthalmic or systemic medications that could affect the pathology condition and patients wearing contact lenses were also excluded from this study. Only tear substitutes without preservatives were accepted as treatment in the selected patients.

### Study design

The development of this study was divided into two phases: Validation 1 and Validation 2. In a first validation (validation 1), the concentrations of these candidate markers was determined in tear samples from 15 control individuals and 27 dry eye patients and 4 blepharitis patients collected by means of the sponge technique (López-Cisternas J et al. Cornea 2006;25:312-8) In the second (validation 2), the same molecules were evaluated in tear samples from 16 control individuals, 20 dry eye patients and 21 with blepharitis patients collected with a microcapillary tube for the purpose of establishing the validity of the markers in patients from different centres, and furthermore for determining if the sample collection method could alter the measurements of the candidate biomarkers. Finally, the discriminatory power of the tested biomarkers for use as a dry eye diagnostic tool was evaluated.

### Tear collection

This research was conducted by medically qualified personnel after approval by Institutional Review Board (IRB) Ethics Committee. Approval was obtained and in strict accordance with the tenets of the Declaration of Helsinki. Informed consent was obtained from all patients after the nature and possible consequences of the study had been explained. For all experiments, tears were collected from the inferior lateral tear meniscus, minimizing irritation of the ocular surface or lid margin. Anaesthetic drops were not instilled.

All tear samples analyzed in the validation 1 were collected using a polyvinyl acetate surgical sponge (Merocel, ref. 0525, Oasis, Glendora, Calif., USA). After collection, the sponge was introduced into a 0.5 ml-tube (Eppendorf, Fremont CA, USA) containing 200 µl of phosphate-buffered saline (PBS) and incubated at 4°C during one hour for protein elution. The tear fluid was subsequently recovered by centrifugation at 15,000 rpm at 4°C for 30 min, and subsequently stored at -80°C until analysis. The tear samples analyzed in the validation 2, were collected by using a 10µl calibrated glass microcapillary tubes (Blaubrand intraMark, Wertheim, Germany) without touching the globe eye or lids and, avoiding any reflex tearing. Patients were requested to position the head slightly reclined in such a way that tears were driven to the most outer side of the lower fornix. After collection, the tear samples were recovered in an eppendorf tube and stored at -80°C until analysis.

The tear samples used in validation assays were quantified by using EZQ protein quantitation kit.

### Validation screening and validation of the candidate biomarkers

Before carrying out the validation by means of sandwich-type ELISA kits of the molecular markers PRDX5, S100A6, CST4, PLAA, ANXA1, ANXA11, GSTP1, GSTA4, GSTM2, SMAD3 and SOD2 (USCN Life Science Inc., Wuhan, China), a screening and fine-tuning assay was performed for each of them. For the performance of this assay, 1:2 serial dilutions of pools of, on one hand, CT samples and, on the other hand, DE samples from 10 to 0.3125 µg of total protein in the manner of a calibration line were loaded. The intention of this assay was to determine which of the candidate markers have a greater discriminatory power to distinguish between the study groups and additionally to choose the minimum amounts of total protein necessary to obtain a maximum signal to noise ratio. Once the screening assays were performed, the validation of the panel of markers with the greatest discriminatory power was performed using individual samples. The ELISA assays were performed following at all times the instructions described by the manufacturer. These validation assays were performed with individual samples using two tear collection methods: the sponge method (Validation 1) and capillary method (Validation 2) to check if the collection methodology used affects the concentrations of the markers in any way.

Once the values of concentration in tears with respect to total protein were obtained for each of the proteins, ROC curve analyses were performed with each of them individually and, additionally, different machine learning approaches such as k-Nearest Neighbour (kNN), Support Vector Machine (SVM), Classification Trees, Random Forest (RF) and Naive Bayes (NB) were performed to assess which of them provide the best accuracy for the correct classification of problem samples. Each algorithm was trained by the biomarker panel and to assess the performance of the learner, a random sampling was carried out with the 70 % of the samples as a training set and the remaining 30 % was used as a test set. This test set was used to evaluate the success of the algorithms for the classification of samples to which the learners have not previously been exposed.

### RESULTS

### Protein Quantification

Total protein quantification was determined in all tear samples by means of EZQ-quantitation kit. In the validation phase, the values of total amount in DE samples collected by sponge and capillary were 28.01 ± 23.38 µg and 29.86 ± 21.11 µg, respectively, showing in both cases significant differences respect to CT group (p-value 0.0023 and 0.0018). As in the discovery phase, these differences were not found in the blepharitis samples, where the amounts of total protein obtained were 45.83 ± 28.86 µg in the case of collection with a sponge and 37.71 ± 23.44 µg with a capillary versus 57.32 ± 46.70 µg and 42.74 ± 38.45 µg of the CT group.

### Validation screening

For the purpose of simultaneously evaluating a panel of candidate biomarkers in tear samples from the same individuals, a preliminary study was performed with the corresponding ELISA kits, the objective of which was to determine those markers with a greater power for discriminating between individuals of the dry eye group versus control individuals and thus reduce the number of candidate markers, such that a joint validation of all of them in the same samples could be carried out.

This approach requires a minimum sample consumption for each of the analytes, being within the linear limits of detection of each of the ELISA kits. To that end, a calibration line was generated using a pool of control samples, as well as a pool of dry eye samples using increasing amounts of sample from 0.3125 to 10 µg. The screening of markers was performed for the molecules ANXA1, PLAA, SOD2, ANXA11, PRDX5, S100A6, GSTP1, GSTM2, CST4, GSTA4 and SMAD3. With the exception of GSTA4 and SMAD3 (data not shown), all of them provided good signal to noise rations and good folds between the dry eye group and the control group (Figure 12). Based on these screening results, where the discriminatory power of the candidate markers was preliminarily evaluated, and bearing in mind the limitations of the protein content of the samples for performing a joint validation of the markers, the proteins ANXA1, PLAA, ANXA11, PRDX5, S100A6 and CST4 were selected for the subsequent validation studies. The proteins GSTP1 and GSTM2 also had a good separation between the control group and the dry eye group. However, the measured absorbances were only distinguished from the background noise by using large amounts of total protein (above 10 µg). Since the objective of the validation assays not only consisted of quantifying the most discriminating markers, but also to do so in the largest possible number of them for each sample, these two markers were discarded. Furthermore, the validation of the protein SOD2 was also discarded due to the high instability in the quantification detected for this protein in the screening assays.

### Validation

The validation studies of the proteins S100A6, CST4, ANXA1, ANXA11, PRDX5 and PLAA were performed by means of sandwich-type ELISA assays (USCN Life Science Inc., Wuhan, China). In a first assay (Validation 1), 15 control samples were evaluated against 27 samples from dry eye individuals and 4 samples from blepharitis individuals, the tear samples being collected in this case with the sponge technique. In a second validation (Validation 2), samples from 16 control individuals, 20 with dry eye pathology and 21 with blepharitis pathology were used, using in this case a capillary as the tear sample collection method.

The results of the validation are shown in Figure 13. In the case of validation 1 (sponge collection), it is observed that the concentration data standardized with respect to the amount of total protein, show a high efficiency in the diagnosis of dry eye pathology using the proteins S100A6, ANXA1 and PLAA, even when used as individual markers with correct assignment percentages of 91.45%, 92.80% and 98.10% respectively (Table 8). According to these data, the marker having the highest sensitivity (96.2%) and specificity (100%) was PLAA. The discriminating analyses performed to determine the power of the variables in a multivariate manner indicated that the molecule which provided the least amount of information in the dry eye classification process was PRDX5 due to the fact that it contained redundant information with respect to that provided by other markers with a greater classification power (data not shown). For this reason this molecule was removed from the second validation of the study. The increase in the PRDX5 protein level observed in the ELISA assays is coincident with the results observed for the isoform A in the Western blot assays of Example 1. The isoform detected in the ELISA assay could be either isoform A or B. However, considering the results shown in Example 2, it is likely that the isoform detected is isoform A.

The results of Validation 2 indicate a trend similar to that found in the case of validation 1 (Figure 13). In this case, all the markers are good markers for the dry eye diagnosis. However, the efficiency obtained in this validation was slightly lower, especially in the case of the annexins, the success percentage decreasing to 70.75 % in the case of Annexin 1 and 78.25% in the case of Annexin 11. In this second validation, the protein S100A6 appears as the best marker to be used individually for dry eye diagnosis with a case success percentage of 81.30%, and it has in turn the highest sensitivity (80.2%), and specificity (82.4%) values jointly (Table 8). These results suggest that significant differences are indeed observed in the concentration of the biomarkers tested according to the method used to collect the tear sample, mainly in the concentrations of annexins. Nevertheless, despite the fact that differences are observed in the concentrations, the diagnostic power provided by the markers is independent of the collection technique used.

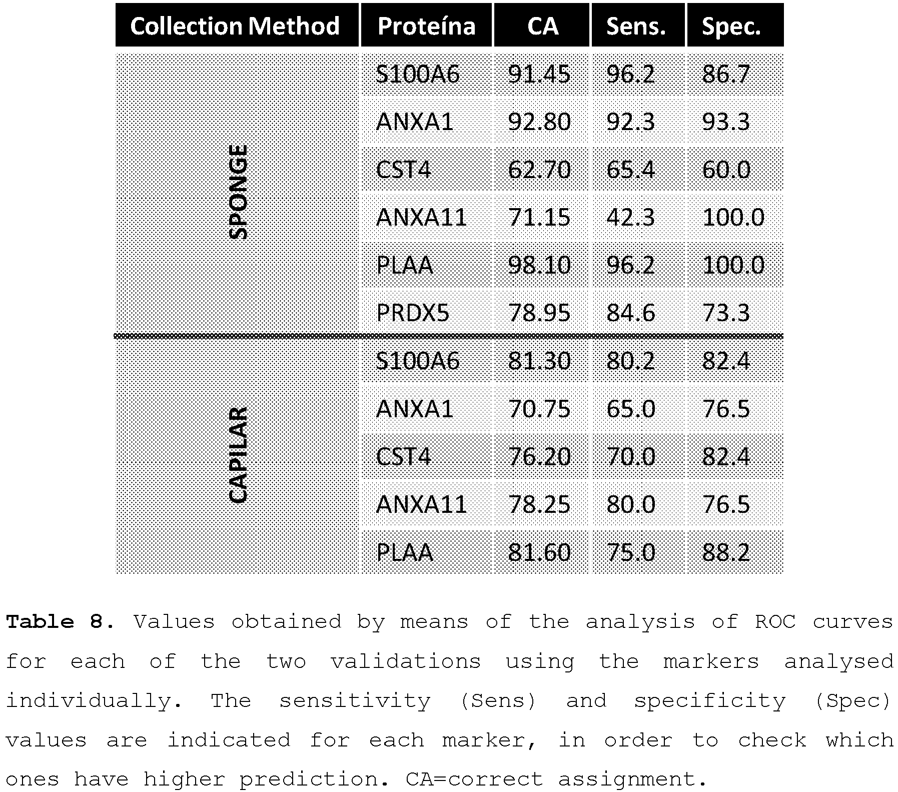

Once the concentration values of each of the markers were obtained individually, the complete panel of 5 markers was used to evaluate the precision thereof in the diagnosis using all the information jointly. To that end, a training of 70% of the data was carried out using different automatic learning algorithms, such as *Naive Bayes, k-Nearest Neighbour* (kNN), *Classification Tree* and *Support Vector Machine,* and a validation of the classification power was subsequently carried out using as a test the remaining 30% of the data, by means of *Random Sampling,* as shown in Table 9. As a result of this analysis in validation 1, the strong classification power obtained is verified by means of using the panel of 5 markers (S100A6, CST4, ANXA1, ANXA11 and PLAA), mainly by means of *Naive Bayes* and *kNN* with high success percentage values or correct assignment values (CA), and therefore a correct classification of the samples tested of 95.83 and 93.75, respectively, and an area under the ROC curve (AUC) of about 0.99 and 0.96, respectively. In the case of validation 2, the best learning algorithm was *Random Forest* with a success percentage of 86.67 and an AUC of 0.90.

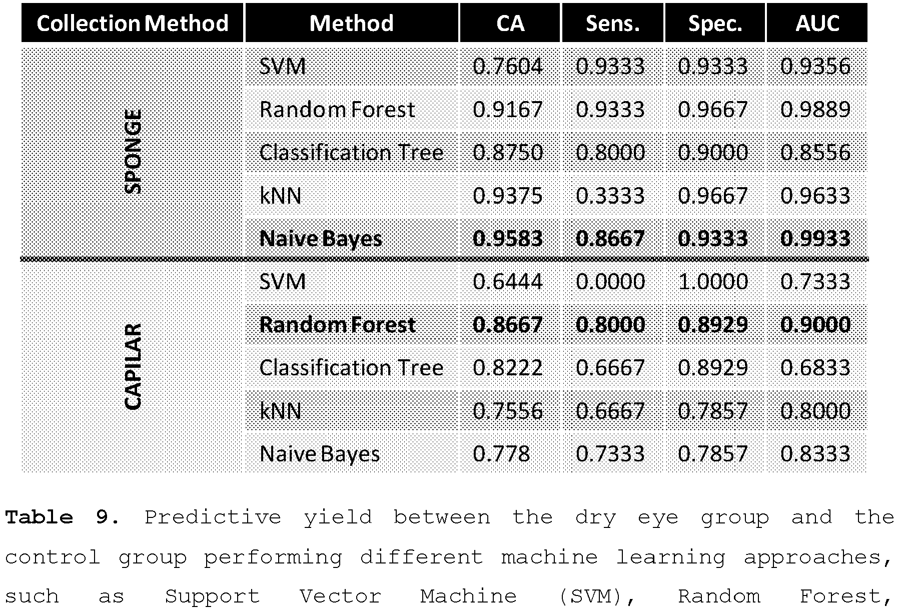

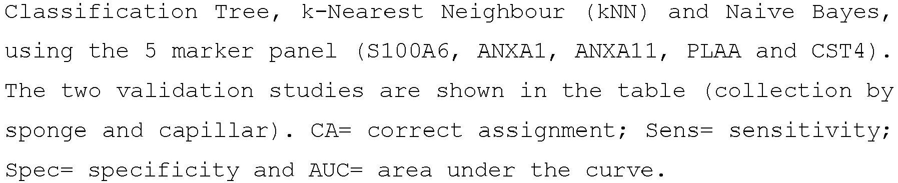

In both validations it was seen that the protein CST4 did not have a high classification power by itself and that during the screening assays it did not either show a very relevant discriminatory power between the dry eye group and the control group. However, when this marker was included within a broader panel, a considerable improvement was achieved in the efficiency of the classification algorithms due to the minimally redundant information of this protein with respect to the rest of the markers of the panel. These results seem to suggest that the classification results obtained with the two sample collection methodologies are comparable, although they seem to be significantly better with the sponge, mainly due to the decrease of the classification power of the two annexins in the case of validation 2.

The S100A6 protein allows distinguishing the control group from the blepharitis group and also the blepharitis group from the dry eye group. Specifically, if the concentration of S100A6 is higher than 115 pg/µg, with respect to a reference sample, the subject may be diagnosed with dry eye or blepharitis. However, if the S100A6 protein concentration is higher than 182 pg/µg with respect to a reference sample, the subject suffers from dry eye. Thus, if the S100A6 protein concentration is higher than 115 pg/µg and lower than 182 pg/µg with respect to a reference sample, the subject suffers from blepharitis (see Figure 14). According to the authors of the invention, if the S100A6 concentration is lower than 115 pg/µg, the probability of the subject belonging to the control group is 80%, while of belonging to the blepharitis group or dry eye group is of 15% and 5%, respectively. On the other hand, if the S100A6 protein concentration is higher than 115 pg/µg and lower than 182 pg/µg with respect to a reference sample, the probability of the subject belonging to the blepharitis group is 71.4%, while the probability of belonging to the dry eye group is 28.6%.

The CST4 (Cystatin 4 or Cystatin S) allows to distinguish the control group from the blepharitis group and also the blepharitis group from the dry eye group. Specifically, if the concentration of CST4 is lower than 118 pg/µg, with respect to a reference sample, the subject may be diagnosed with dry eye or blepharitis. However, if the concentration of CST4 is lower than 98.5 pg/µg, with respect to a reference sample, the subject suffers from dry eye. Thus, it the CST4 protein concentration is higher than 98.5 pg/µg but lower than 118 pg/µg, the subject suffers from blepharitis (see Figure 15).

The CST1 (Cystatin 1 or Cystatin N) allows to distinguish the control group from the blepharitis group, the control group from the dry eye group and also the blepharitis group from the dry eye group. Specifically, if the concentration of CST1 is higher than 12.4 pg/µg, with respect to a reference sample, the subject suffers from blepharitis. However, if the concentration of CST1 is lower than 7 pg/µg, with respect to a reference sample, the subject suffers from dry eye (see Figure 16).

The Galectin 7 allows the distinction between the control group and the blepharitis group and also the control group and the dry eye group. It is thus possible to distinguish between the dry eye group and the blepharitis group by determining the Galectin 7 protein concentration. If the concentration of Galectin 7 is lower than 257 pg/µg, with respect to the reference sample, the subject suffers from dry eye. However, if said concentration is lower than 190 pg/µg, with respect to the reference sample, the subject suffers from blepharitis. Thus, to distinguish between dry eye and blepharitis, the galectin 7 concentration is lower than 190 pg/µg, with respect to the reference sample, if the subject suffers from blepharitis and if said concentration is between 190 pg/µg and 257 pg/µg, the subject suffers from dry eye (Figure 17).

The JNK proteins (isoforms JNK 1, 2 and 3) allows to distinguish the control group from the blepharitis group, as well as the control group from the dry eye group. Specifically, if the JNK protein concentration is higher than the protein concentration in the reference sample, it is indicative of the subject suffering from dry eye or blepharitis (Figure 18).

## Claims

1. An *in vitro* method for diagnosing dry eye in a subject which comprises:
(i) determining the level of S100A6 protein in a tear sample from said subject, and
(ii) comparing the level of said protein with the level of said protein in a reference sample,
wherein if the subject has dry eye, the level of S100A6 protein is elevated with respect to its level in the reference sample.

2. The method according to claim 1, wherein the level of one or more proteins selected from the group of PLAA, Serpin B13, Reticulocalbin 1, Superoxide dismutase, SMAD3, GSTM2, GSTA4, Cystatin S and SSB is additionally determined.

3. A kit comprising at least one reagent for the detection of protein S100A6 for its use in the *in vitro* method for diagnosing dry eye in a subject according to claim 1, said kit additionally comprising reagents for the detection of one or more proteins selected from the group of Serpin B13, Reticulocalbin 1, Superoxide dismutase, SMAD3, GSTM2, GSTA4 and SSB.

4. A kit for use according to claim 3, further comprising a positive control.

## Patentansprüche

1. *In vitro* Verfahren zur Diagnose von trockenem Auge bei einem Subjekt, wobei das Verfahren die Schritte umfasst, bei denen man:
(i) die Menge des S100A6 Proteins in einer Tränenprobe eines Subjekts bestimmt; und
(ii) die Menge dieses Proteins mit der Menges des Proteins in einer Referenzprobe vergleicht,
wobei die Menge des S100A6-Proteins im Vergleich zu der Menge in der Referenzprobe erhöht ist, wenn das Subjekt ein trockenes Auge aufweist.

2. Verfahren gemäß Anspruch 1, wobei ferner die Menge von einem oder mehreren Proteinen bestimmt wird, die aus der Gruppe bestehend aus PLAA, Serpin B13, Reticulocalbin 1, Superoxiddismutase, SMAD3, GSTM2, GSTA4, Cystatin S und SSB ausgewählt wurden.

3. Kit zur Verwendung in einem *in vitro* Verfahren zur Diagnose von trockenem Auge in einem Subjekt gemäß Anspruch 1, welches mindestens ein Reagenz zum Nachweis des Proteins S100A6 umfasst, wobei das Kit ferner Reagenzien zum Nachweis von einem oder mehreren der Proteine umfasst, die aus der Gruppe bestehend aus Serpin B13, Reticulocalbin 1, Superoxiddismutase, SMAD3, GSTM2, GSTA4 und SSB ausgewählt wurden.

4. Kit zur Verwendung gemäß Anspruch 3, welches ferner eine Positivkontrolle umfasst.

## Revendications

1. Un procédé *in vitro* pour diagnostiquer la sécheresse oculaire chez un sujet, qui comprend :
(i) déterminer le taux de protéine S100A6 dans un échantillon de larme prélevé chez ledit patient, et
(ii) comparer le taux de ladite protéine avec le taux de ladite protéine dans un échantillon de référence,
où, si le patient est atteint de sécheresse oculaire, le taux de protéine S100A6 est élevé par rapport à son taux dans l'échantillon de référence.

2. Le procédé selon la revendication 1, où le taux d'une ou de plusieurs protéines sélectionnées parmi le groupe de PLAA, Serpine B13, Réticulocalbine 1, Superoxyde dismutase, SMAD3, GSTM2, GSTA4, Cystatine S et SSB est par ailleurs déterminé.

3. Un kit comprenant au moins un réactif pour la détection de la protéine S100A6 pour son utilisation dans le procédé *in vitro* pour diagnostiquer la sécheresse oculaire chez un sujet selon la revendication 1, ledit kit comprenant en outre des réactifs pour la détection d'une ou de plusieurs protéines sélectionnées parmi le groupe constitué de Serpine B13, Réticulocalbine 1, Superoxyde dismutase, SMAD3, GSTM2, GSTA4 et SSB.

4. Un kit pour une utilisation selon la revendication 3, comprenant en outre un témoin positif.
